# EUROPÄISCHE PATENTSCHRIFT

(11) **EP 2 046 785 B1**
(45) Veröffentlichungstag und Bekanntmachung des Hinweises auf die Patenterteilung: **27.02.2013**
(21) Anmeldenummer: 07765072.9
(22) Anmeldetag: 05.07.2007
(51) Int. Cl.: C07D 409/10

(54) **1,4-BIS(2-THIENYLVINYL)BENZOLDERIVATE UND IHRE VERWENDUNG**
1,4-BIS(2-THIENYLVINYL)BENZOL DERIVATIVES AND THEIR USE
DERIVES DU 1,4-BIS(2-THIENYLVINYL)BENZENE ET LEUR UTILISATION

(30) Priorität: 28.07.2006 DE 102006035041
(43) Veröffentlichungstag der Anmeldung: 15.04.2009
(73) Patentinhaber: Merck Patent GmbH, 64293 Darmstadt (DE)
(72) Erfinder: BUESING, Arne, 65929 Frankfurt am Main (DE); HEUN, Susanne, 65812 Bad Soden (DE); LUDEMANN, Aurélie, 60322 Frankfurt (DE); MEYER, Frank, 69120 Heidelberg (DE); SCHULTE, Niels, 65779 Kelkheim (DE)
(86) Internationale Anmeldenummer: PCT/EP2007/005950
(87) Internationale Veröffentlichungsnummer: WO 2008/011967

(56) Entgegenhaltungen:
- WO-A-03/016430
- WO-A-2004/050794
- WO-A-2005/030827
- JORGENSEN, MIKKEL ET AL: "Stepwise Unidirectional Synthesis of Oligo Phenylene Vinylenes with a Series of Monomers. Use in Plastic Solar Cells" JOURNAL OF ORGANIC CHEMISTRY , 70(15), 6004-6017 CODEN: JOCEAH; ISSN: 0022-3263, 2005, XP002458010

## Beschreibung

Die vorliegende Erfindung betrifft neue 1,4-Bis(2-thienylvinyl)-benzolderivate, diese enthaltende konjugierte Polymere, Dendrimere, Blends, Mischungen und Formulierungen, sowie ihre Verwendung in elektronischen Vorrichtungen, insbesondere in polymeren organischen Leuchtdioden.

Konjugierte Polymere werden zur Zeit intensiv als vielversprechende Materialien in PLEDs (Polymer Light Emitting Diodes) untersucht. Ihre einfache Verarbeitung im Gegensatz zu SMOLEDs (Small Molecule Organic Light Emitting Diodes) verspricht eine kostengünstigere Herstellung von entsprechenden Leuchtdioden.

Da PLEDs meist nur aus einer lichtemittierenden Schicht bestehen, werden Polymere benötigt, die sämtliche Funktionen einer OLED (Ladungsinjektion, Ladungstransport, Rekombination) in sich vereinigen können. Daher werden während der Polymerisation unterschiedliche Monomere eingesetzt, die die entsprechenden Funktionen übernehmen. So ist es für die Erzeugung aller drei Emissionsfarben in der Regel nötig, bestimmte Comonomere in die entsprechenden Polymere einzupolymerisieren (vgl. z.B. WO 00/046321, WO 03/020790 und WO 02/077060). So ist beispielsweise ausgehend von einem blau emittierenden Grundpolymer ("backbone") die Erzeugung der beiden anderen Primärfarben Rot und Grün möglich.

SMOLED sind im Gegensatz zu Polymeren aus mehreren Schichten aufgebaut, welche die verschiedenen Funktionen erfüllen. Auch hier tritt eine lichtemittierende Schicht auf, welche den Emitter enthält.

Die wichtigsten Kriterien einer OLED sind Effizienz, Farbe und Lebensdauer. Diese Eigenschaften werden maßgeblich durch den/die Emitter bestimmt. PLEDs werden zwar unter Verwendung von Emittern aufgebaut, allerdings bleiben Lebensdauer und Effizienz noch immer wesentlich hinter den Anforderungen für eine Verwendung in großflächigen Displays zurück. Auch die aus dem Stand der Technik bekannten Materialien für PLEDS weisen häufig Mängel in Bezug auf die Parameter Lebensdauer, Effizienz und Farbe auf.

Überraschenderweise wurde festgestellt, dass lumineszierende Strukturen, insbesondere Polymere und Dendrimere, enthaltend Derivate des 1,4-Bis(2-thienylvinyl)benzols mit Arylsubstituenten sehr hohe Effizienzen zeigen und die Lebensdauern um mehrere Größen-ordnungen im Vergleich zu bisherigen Referenzsystemen erhöhen.

In der WO 2005/030827 A1 werden weiße Licht emittierende Polymere beschrieben. Auf Seite 9 werden als grün emittierende Comonomere unter anderem Vinylaryleneinheiten einer allgemeinen Formel IX 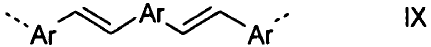 vorgeschlagen, worin Ar ein optional substituiertes, aromatisches oder heteroaromatisches Ringsystem ist. Dabei sollen diese Einheiten mindestens ein elektronenreiches oder elektronenreiche Substituenten aufweisendes Ringsystem Ar enthalten, wie Thiophen, Furan, Pyrrol oder mit Alkoxy-, Aryloxy- oder Aminogruppen substituiertes Phenyl. Die konkreten Beispiele der WO 2005/030827 A1 offenbaren auf Seite 17 ein Monomer der Formel M6 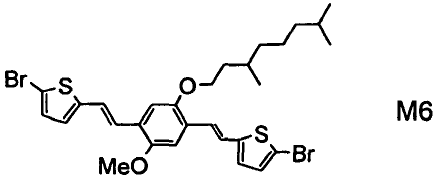 mit zwei Thiophen-2,5-diylgruppen und einer 1,5-Dialkoxy-1,4-phenylgruppe. Die Verbindungen der vorliegenden Erfindung werden darin jedoch nicht offenbart.

Die Vinylarylenverbindungen aus dem Stand der Technik weisen jedoch einige Nachteile auf, wie z.B. eine erhöhte Oxidationsempfindlichkeit in Lösung. Des weiteren hat sich gezeigt, dass Alkoxysubstituenten oft nicht stabil gegenüber Lochtransport sind. Außerdem ist die Aufreinigung des Monomers/Polymers durch die erhöhte Oxidationsempfindlichkeit erschwert. Darüber hinaus zeigen die Polymere aus dem Stand der Technik oft eine Verschiebung der Emissionsfarbe zu Gelb.

Aufgabe der vorliegenden Erfindung war daher unter anderem die Bereitstellung von Derivaten des 1,4-Bis(2-thinylvinyl)benzols, welche die oben genannten Nachteile nicht oder nur in geringerem Ausmaß zeigen. Diese Aufgabe wurde erfindungsgemäß gelöst durch Bereitstellung von Verbindungen gemäß Anspruch 1.

Gegenstand der Erfindung sind somit 1,4-Bis(2-thienylvinyl)benzolderivate der Formel I 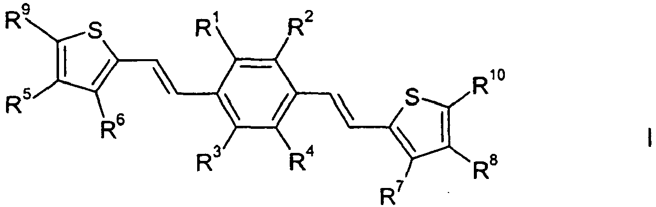 wobei die verwendeten Symbole und Indices folgende Bedeutung besitzen:
- R¹ bis R¹⁰: sind unabhängig voneinander H, X¹, X¹-Sp-, -CN, -NO₂, -NCS, -NCO, -OCN, -SCN, -SF₅, -Si(R)₃ oder eine geradkettige, verzweigte oder cyclische Alkyl- oder Alkoxygruppe mit 1 bis 22 C-Atomen, in der auch ein oder mehrere nicht benachbarte C-Atome durch -C(R)=C(R)-, -C≡C-, -N(R)-, -O-, -S-, -CO-, -CO-O-, -O-CO-, -O-CO-O- ersetzt sein können, wobei auch ein oder mehrere H-Atome durch Fluor ersetzt sein können, eine Aryl-, Aryloxy-, Heteroaryl- oder Heteroaryloxygruppe mit 5 bis 40 C-Atomen, welche auch durch ein oder mehrere nicht-aromatische Reste R¹⁻¹⁰ substituiert sein kann, wobei auch zwei oder mehrere der Reste R¹⁻¹⁰ miteinander ein aliphatisches oder aromatisches, mono- oder polycyclisches Ringsystem bilden können, welches auch mit dem Benzolring oder den Thiophenringen in Formel I ein kondensiertes Ringsystem bilden kann, oder bedeuten eine Verknüpfung im Polymer,
- R: ist bei jedem Auftreten gleich oder verschieden H, Halogen, eine geradkettige, verzweigte oder cyclische Alkylkette mit 1 bis 22 C-Atomen, in der auch ein oder mehrere nicht benachbarte C-Atome durch -O-, -S-, -CO-, -CO-O-, -O-CO-, -O-CO-O-ersetzt sein können, wobei auch ein oder mehrere H-Atome durch Fluor ersetzt sein können, eine Aryl-, Aryloxy-, Heteroaryl- oder Heteroaryloxygruppe mit 5 bis 40 C-Atomen, welche auch durch ein oder mehrere nicht-aromatische Reste R¹⁻¹⁰ substituiert sein kann; dabei können auch mehrere Reste R miteinander und/oder mit weiteren Resten R¹⁻¹⁰ ein aromatisches oder aliphatisches, mono- oder polycyclisches Ringsystem bilden, welches auch mit dem Benzolring oder den Thiophenringen in Formel I ein kondensiertes Ringsystem bilden kann,
- X¹: ist bei jedem Auftreten gleich oder verschieden eine reaktive Gruppe, ausgewählt aus Halogen, -OH, -COOH, -CY¹O, -CHO, -CH₂Cl, -N(R⁰)₂, -Si(R⁰)₃, -Sn(R⁰)₃, -B(R⁰)₂, -B(OR⁰)₂, -B(OH)₂, - CR⁰=C(R⁰)₂, -C≡CH, -O-SO₂R⁰, -SiMe₂F oder -SiMeF₂, worin Y¹ Halogen, Me Methyl und R⁰ Alkyl oder Aryl bedeutet, wobei auch zwei Gruppen R⁰ ein aromatisches oder aliphatisches, mono- oder polycyclisches Ringsystem bilden können, oder ausgewählt aus CH₂=CW¹-COO-, CH₂=CW¹-CO-, 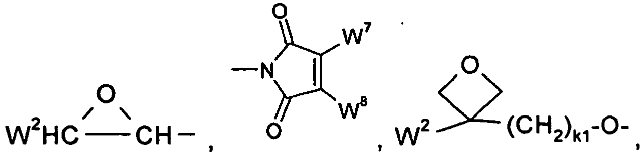 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-,
CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-, und W⁴W⁵W⁶Si-, worin W¹ H, F, Cl, CN, CF₃, Phenyl oder Alkyl mit 1 bis 5 C-Atomen bedeutet, W² und W³ jeweils unabhängig voneinander H oder Alkyl mit 1 bis 5 C-Atomen bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet und k₁ und k₂ jeweils unabhängig voneinander 0 oder 1 bedeuten,
- Sp: ist bei jedem Auftreten gleich oder verschieden eine Abstandsgruppe oder eine Einfachbindung, wobei die Abstandsgruppe Sp ausgewählt ist aus der Formel Sp'-X', so dass "X'-Sp-" "X¹-Sp'-X'-" bedeutet, wobei
- Sp': Alkylen mit 1 bis 20 C-Atomen bedeutet, welches durch F, Cl, Br, I oder CN ein- oder mehrfach substituiert sein kann, und worin auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-, -NR⁰-CO-O-, -O-CO-NR⁰-, -NR⁰-CO-NR⁰-, -CH=CH- oder -C≡C- ersetzt sein können, dass O- und/oder S-Atome nicht direkt miteinander verknüpft sind,
- X': -O-, -S-, -CO-, -COO-, -OCO-, -O-COO-, -CO-NR⁰-, -NR⁰-CO-, -NR⁰-CO-NR⁰-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-COO-, -OCO-CH=CH- oder eine Einfachbindung bedeutet,
- R⁰ und R⁰⁰: jeweils unabhängig voneinander H oder Alkyl mit 1 bis 12 C-Atomen bedeuten, und
- Y² und Y³: jeweils unabhängig voneinander H, F, Cl oder CN bedeuten,
worin mindestens einer der Reste R¹ bis R⁴ eine Arylgruppe bedeutet, welche durch ein oder mehrere nicht-aromatische Reste R¹⁻¹⁰ substituiert sein kann, wobei auch zwei oder mehrere der Reste R¹⁻¹⁰ miteinander ein aliphatisches oder aromatisches, mono- oder polycyclisches Ringsystem bilden können, welches auch mit dem Benzolring oder den Thiophenringen in Formel I ein kondensiertes Ringsystem bilden kann.

Ein weiterer Gegenstand der vorliegenden Erfindung sind Polymere oder Dendrimere, vorzugsweise konjugierte Polymere oder Dendrimere, erhältlich durch Polymerisation einer oder mehrerer Verbindungen der Formel I, optional mit zusätzlichen Comonomeren.

Ein weiterer Gegenstand der vorliegenden Erfindung ist eine Mischung, vorzugsweise bestehend aus niedermolekularen Komponenten, enthaltend eine oder mehrere Verbindungen der Formel I und zusätzlich eine oder mehrere lichtemittierende und/oder polymerisierbare Verbindungen.

Ein weiterer Gegenstand der vorliegenden Erfindung ist ein Polymer Blend enthaltend ein oder mehrere erfindungsgemäße Polymere oder Dendrimere, sowie optional enthaltend eine oder mehrere weitere polymere, oligomere, dendritische oder niedermolekulare Substanzen.

Ein weiterer Gegenstand der vorliegenden Erfindung ist eine Formulierung enthaltend
- eine oder mehrere Verbindungen der Formel I oder aus diesen erhältliche Polymere, Dendrimere, Polymer Blends oder niedermolekulare Mischungen,
- ein oder mehrere Lösungsmittel und/oder ein oder mehrere polymere Bindemittel.

Ein weiterer Gegenstand der vorliegenden Erfindung ist die Verwendung der erfindungsgemäßen Verbindungen, Polymere, Dendrimere, Mischungen und Formulierungen in elektronischen oder elektrooptischen Vorrichtungen, insbesondere in organischen bzw. polymeren organischen Leuchtdioden (OLED, PLED), organischen Feld-Effekt-Transistoren (OFETs), organischen integrierten Schaltungen (O-ICs), organischen Dünnfilmtransistoren (TFTs), organischen Solarzellen (O-SCs), organischen Laserdioden (O-Laser), organischen photovoltaischen (OPV) Elementen oder Vorrichtungen oder organischen Photorezeptoren (OPCs).

Die Begriffe "Alkyl", "Aryl", "Heteroaryl" etc. umfassen auch mehrbindige Gruppen, beispielsweise Alkylen, Arylen, Heteroarylen etc..

Der Begriff "Kohlenstoffrest" bedeutet vor- und nachstehend einen ein-oder mehrbindigen organischen Rest enthaltend mindestens ein Kohlenstoffatom, wobei dieser entweder keine weiteren Atome enthält (wie z.B. -C≡C-), oder gegebenenfalls ein oder mehrere weitere Atome wie beispielsweise N, O, S, P, Si, Se, As, Te oder Ge enthält (z.B. Carbonyl etc.). Der Begriff "Kohlenwasserstoffrest" bedeutet einen Kohlenstoffrest, der zusätzlich ein oder mehrere H-Atome und gegebenenfalls ein oder mehrere Heteroatome wie beispielsweise N, O, S, P, Si, Se, As, Te oder Ge enthält.

Der Begriff "Aryl" bedeutet eine aromatische Kohlenstoffgruppe oder eine davon abgeleitete Gruppe. Der Begriff "Heteroaryl" bedeutet "Aryl" gemäß vorstehender Definition, enthaltend ein oder mehrere Heteroatome.

Ein Kohlenstoff- oder Kohlenwasserstoffrest kann eine gesättigte oder ungesättigte Gruppe sein. Ungesättigte Gruppen sind beispielsweise Aryl-, Alkenyl- oder Alkinylgruppen. Ein Kohlenstoff- oder Kohlenwasserstoffrest mit mehr als 3 C-Atomen kann geradkettig, verzweigt und/oder cyclisch sein, und kann auch Spiroverknüpfungen oder kondensierte Ringe aufweisen.

Bevorzugte Kohlenstoff- und Kohlenwasserstoffreste sind gegebenenfalls substituiertes Alkyl, Alkenyl, Alkinyl, Alkoxy, Alkylcarbonyl, Alkoxycarbonyl, Alkylcarbonyloxy und Alkoxycarbonyloxy mit 1 bis 40, vorzugsweise 1 bis 25, besonders bevorzugt 1 bis 18 C-Atomen, gegebenenfalls substituiertes Aryl oder Aryloxy mit 6 bis 40, vorzugsweise 6 bis 25 C-Atomen, oder gegebenenfalls substituiertes Alkylaryl, Arylalkyl, Alkylaryloxy, Arylalkyloxy, Arylcarbonyl, Aryloxycarbonyl, Arylcarbonyloxy und Aryloxycarbonyloxy mit 6 bis 40, vorzugsweise 6 bis 25 C-Atomen.

Weitere bevorzugte Kohlenstoff- und Kohlenwasserstoffreste sind C₁-C₄₀ Alkyl, C₂-C₄₀ Alkenyl, C₂-C₄₀ Alkinyl, C₃-C₄₀ Allyl, C₄-C₄₀ Alkyldienyl, C₄-C₄₀ Polyenyl, C₆-C₄₀ Aryl, C₆-C₄₀ Alkylaryl, C₆-C₄₀ Arylalkyl, C₆-C₄₀ Alkylaryloxy, C₆-C₄₀ Arylalkyloxy, C₆-C₄₀ Heteroaryl, C₄-C₄₀ Cycloalkyl, C₄-C₄₀ Cycloalkenyl, etc.. Besonders bevorzugt sind C₁-C₂₂ Alkyl, C₂-C₂₂ Alkenyl, C₂ -C₂₂ Alkinyl, C₃-C₂₂ Allyl, C₄-C₂₂ Alkyldienyl, C₆-C₁₂ Aryl, C₆-C₂₀ Arylalkyl und C₆-C₂₀ Heteroaryl.

Weitere bevorzugte Kohlenstoff- und Kohlenwasserstoffreste sind geradkettige, verzweigte oder cyclische Alkylreste mit 1 bis 40, vorzugsweise 1 bis 22 C-Atomen, welche unsubstituiert oder durch F, Cl, Br, I oder CN ein- oder mehrfach substituiert sind, und worin ein oder mehrere nicht benachbarte CH₂-Gruppen jeweils unabhängig voneinander durch -C(R)=C(R)-, -C≡C-, -N(R)-, -O-, -S-, -CO-, -CO-O-, -O-CO-, -O-CO-O- so ersetzt sein können, dass O- und/oder S-Atome nicht direkt miteinander verknüpft sind, wobei R die oben angegebene Bedeutung hat.

Bevorzugte Alkylgruppen sind beispielsweise Methyl, Ethyl, n-Propyl, Isopropyl, n-Butyl, Isobutyl, s-Butyl, t-Butyl, 2-Methylbutyl, n-Pentyl, s-Pentyl, Cyclopentyl, n-Hexyl, Cyclohexyl, 2-Ethylhexyl, n-Heptyl, Cycloheptyl, n-Octyl, Cyclooctyl, Dodecanyl, Trifluoromethyl, Perfluoro-n-butyl, 2,2,2-Trifluoroethyl, Perfluorooctyl, Perfluorohexyl etc..

Bevorzugte Alkenylgruppen sind beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl etc..

Bevorzugte Alkinylgruppen sind beispielsweise Ethinyl, Propinyl, Butinyl, Pentinyl, Hexinyl, Octinyl etc..

Bevorzugte Alkoxygruppen sind beispielsweise Methoxy, Ethoxy, 2-Methoxyethoxy, n-Propoxy, i-Propoxy, n-Butoxy, i-Butoxy, s-Butoxy, t-Butoxy, 2-Methylbutoxy, n-Pentoxy, n-Hexoxy, n-Heptoxy, n-Octoxy etc..

Bevorzugte Aminogruppen sind beispielsweise Dimethylamino, Methylamino, Methylphenylamino, Phenylamino, etc..

Arylgruppen können monocyclisch oder polycyclisch sein, d.h. sie können einen Ring (beispielsweise Phenyl) oder zwei oder mehr Ringe aufweisen, welche auch kondensiert (beispielsweise Naphthyl) oder kovalent verknüpft sein können (beispielsweise Biphenyl), oder eine Kombination von kondensierten und verknüpften Ringen beinhalten. Bevorzugt sind vollständig konjugierte Arylgruppen.

Bevorzugte Arylgruppen sind beispielsweise Phenyl, Biphenyl, Triphenyl, [1,1':3',1"]Terphenyl-2'-yl, Naphthyl, Anthracen, Binaphthyl, Phenanthren, Pyren, Dihydropyren, Chrysen, Perylen, Tetracen, Pentacen, Benzpyren, Fluoren, Inden, Indenofluoren, Spirobifluoren, etc..

Bevorzugte Heteroarylgruppen sind beispielsweise 5-gliedrige Ringe wie Pyrrol, Pyrazol, Imidazol, 1,2,3-Triazol, 1,2,4-Triazol, Tetrazol, Furan, Thiophen, Selenophen, Oxazol, Isoxazol, 1,2-Thiazol, 1,3-Thiazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 6-gliedrige Ringe wie Pyridin, Pyridazin, Pyrimidin, Pyrazin, 1,3,5-Triazin, 1,2,4-Triazin, 1,2,3-Triazin, 1,2,4,5-Tetrazin, 1,2,3,4-Tetrazin, 1,2,3,5-Tetrazin, oder kondensierte Gruppen wie Indol, Isoindol, Indolizin, Indazol, Benzimidazol, Benzotriazol, Purin, Naphthimidazol, Phenanthrimidazol, Pyridimidazol, Pyrazinimidazol, Chinoxalinimidazol, Benzoxazol, Naphthoxazol, Anthroxazol, Phenanthroxazol, Isoxazol, Benzothiazol, Benzofuran, Isobenzofuran, Dibenzofuran, Chinolin, Isochinolin, Pteridin, Benzo-5,6-chinolin, Benzo-6,7-chinolin, Benzo-7,8-chinolin, Benzoisochinolin, Acridin, Phenothiazin, Phenoxazin, Benzopyridazin, Benzopyrimidin, Chinoxalin, Phenazin, Naphthyridin, Azacarbazol, Benzocarbolin, Phenanthridin, Phenanthrolin, Thieno[2,3b]thiophen, Thieno[3,2b]thiophen, Dithienothiophen, Isobenzothiophen, Dibenzothiophen, Benzothiadiazothiophen, oder Kombinationen dieser Gruppen. Die Heteroarylgruppen können auch mit Alkyl, Alkoxy, Thioalkyl, Fluor, Fluoralkyl oder weiteren Aryl- oder Heteroarylgruppen substituiert sein.

Die Aryl-, Heteroaryl-, Kohlenstoff- und Kohlenwasserstoffreste weisen gegebenenfalls einen oder mehrere Substituenten auf, welche vorzugsweise ausgewählt sind aus der Gruppe enthaltend Silyl, Sulfo, Sulfonyl, Formyl, Amin, Imin, Nitril, Mercapto, Nitro, Halogen, C₁₋₁₂ Alkyl, C₆₋₁₂ Aryl, C₁₋₁₂ Alkoxy, Hydroxy oder Kombinationen dieser Gruppen.

Bevorzugte Substituenten sind beispielsweise löslichkeitsfördernde Gruppen wie Alkyl oder Alkoxy, elektronenziehende Gruppen wie Fluor, Nitro oder Nitril, oder Substituenten zur Erhöhung der Glastemperatur (Tg) im Polymer, insbesondere voluminöse Gruppen wie z.B. t-Butyl oder gegebenenfalls substituierte Arylgruppen.

Besonders bevorzugte Substituenten, im Folgenden auch als "L" bezeichnet, sind beispielsweise F, Cl, Br, I, -CN, -NO₂, -NCO, -NCS, -OCN, -SCN, -C(=O)N(R)₂, -C(=O)Y¹, -C(=O)R, -N(R)₂, worin R die oben angegebene Bedeutung hat und Y¹ Halogen bedeutet, Silyl, Aryl mit 4 bis 40, vorzugsweise 6 bis 20 C Atomen, und geradkettiges oder verzweigtes Alkyl, Alkoxy, Alkylcarbonyl, Alkoxycarbonyl, Alkylcarbonyloxy oder Alkoxycarbonyloxy mit 1 bis 22 C-Atomen, worin ein oder mehrere H-Atome gegebenenfalls durch F oder Cl ersetzt sein können.

In einer ersten bevorzugten Ausführungsform bedeutet X¹ eine Gruppe, die für eine Polymerisationsreaktion unter C-C-, C-N- oder C-O-Verknüpfung, wie beispielsweise die untenstehenden Polymerisationen gemäß SUZUKI, YAMAMOTO, STILLE, Buchwald, Kumada oder Sonogashira, geeignet ist.

Die Gruppen X¹ dieses ersten Typs sind Halogen, insbesondere Br, Cl oder I, ferner -OH, -COOH, -CY¹O, -CHO, -CH₂Cl, -N(R⁰)₂, -Si(R⁰)₃, - Sn(R⁰)₃, -B(R⁰)₂, -B(OR⁰)₂, -B(OH)₂, -CR⁰=C(R⁰)₂, -C≡CH, -O-SO₂R⁰, -SiMe₂F oder -SiMeF₂, worin Y¹ Halogen, Me Methyl und R⁰ optional substituiertes Alkyl oder Aryl bedeutet, wobei auch zwei Gruppen R⁰ ein aromatisches oder aliphatisches, mono- oder polycyclisches Ringsystem bilden können. Bevorzugte Gruppen -O-SO₂R⁰ sind insbesondere O-Tosylat, O-Triflat, O-Mesylat und O-Nonaflat.

In einer zweiten bevorzugten Ausführungsform bedeutet X¹ eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine C-C-Doppelbindung oder C-C-Dreifachbindung, sowie zur Polymerisation unter Ringöffnung geeignete Gruppen wie beispielsweise Oxetan- oder Epoxygruppen.

Die Gruppen X¹ dieses zweiten Typs sind ausgewählt aus CH₂=CW¹-COO-, CH₂=CW¹-CO-, 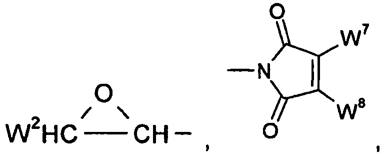 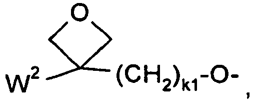 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-, und W⁴W⁵W⁶Si-, worin W¹ H, F, Cl, CN, CF₃, Phenyl or Alkyl mit 1 bis 5 C-Atomen, insbesondere H, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, welches optional mit ein oder mehreren Resten L wie oben definiert ist, und k₁ und k₂ jeweils unabhängig voneinander 0 oder 1 bedeuten.

Bevorzugte Gruppen X¹ dieses zweiten Typs sind CH₂=CH-COO-, CH₂=C(CH₃)-COO-, CH₂=CH-, CH₂=CH-O-, (CH₂=CH)₂CH-OCO-, (CH₂=CH)₂CH-O-, 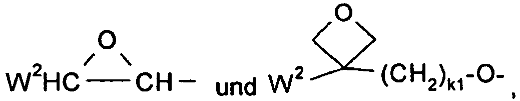 insbesondere Vinyl, Acrylat, Methacrylat, Oxetan und Epoxy.

Der Begriff "Abstandsgruppe" (engl. "spacer" oder "spacer group") (Sp) ist dem Fachmann bekannt und in der Literatur beschrieben, siehe beispielsweise Pure Appl. Chem. 73(5), 888 (2001).

Die Abstandsgruppen Sp sind ausgewählt aus der Formel Sp'-X', so dass "X¹-Sp-" "X¹-Sp'-X'-" bedeutet, wobei
- Sp': Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-, -NR⁰-CO-O-, -O-CO-NR⁰-, -NR⁰-CO-NR⁰-, -CH=CH- oder -C≡C- ersetzt sein können, dass O- und/oder S-Atome nicht direkt miteinander verknüpft sind,
- X': -O-, -S-, -CO-, -COO-, -OCO-, -O-COO-, -CO-NR⁰-, -NR⁰-CO-, -NR⁰-CO-NR⁰-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-COO-, -OCO-CH=CH- oder eine Einfachbindung bedeutet,
- R⁰ und R⁰⁰: jeweils unabhängig voneinander H oder Alkyl mit 1 bis 12 C-Atomen bedeuten, und
- Y² und Y³: jeweils unabhängig voneinander H, F, Cl oder CN bedeuten.
- X': ist vorzugsweise -O-, -S-, -CO-, -COO-, -OCO-, -O-COO-, -CO-NR⁰-, -NR⁰-CO-, -NR⁰-CO-NR⁰- oder eine Einfachbindung.

Typische Abstandsgruppen Sp' sind beispielsweise -(CH₂)ₚ-, -(CH₂CH₂O)_{q}-CH₂CH₂-, -CH₂CH₂-S-CH₂CH₂-, -CH₂CH₂-NH-CH₂CH₂- oder -(SiR⁰R⁰⁰-O)ₚ, worin p eine ganze Zahl von 2 bis 12 ist, q eine ganze Zahl von 1 bis 3 ist, und R⁰ und R⁰⁰ die oben angegebenen Bedeutungen besitzen.

Besonders bevorzugte Gruppen Sp' sind beispielsweise jeweils geradkettiges Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyliminoethylen, 1-Methylalkylen, Ethenylen, Propenylen und Butenylene.

### "Halogen" bedeutet F, Cl, Br oder I.

"Konjugierte Polymere" im Sinne dieser Erfindung sind Polymere, die in der Hauptkette hauptsächlich sp²-hybridisierte (bzw. gegebenenfalls auch sp-hybridisierte) Kohlenstoffatome, die auch durch entsprechende Heteroatome ersetzt sein können, enthalten. Dies bedeutet im einfachsten Fall abwechselndes Vorliegen von Doppel- und Einfachbindungen in der Hauptkette, aber auch Polymere mit Einheiten wie beispielsweise metaverknüpftes Phenylen sollen im Sinne dieser Erfindung als konjugierte Polymere gelten. "Hauptsächlich" meint, dass natürlich (unwillkürlich) auftretende Defekte, die zu Konjugationsunterbrechungen führen, den Begriff "konjugiertes Polymer" nicht entwerten. Des Weiteren wird in diesem Anmeldetext ebenfalls als "konjugiert" bezeichnet, wenn sich in der Hauptkette beispielsweise Arylamineinheiten, Arylphosphineinheiten und/oder bestimmte Heterocyclen (d.h. Konjugation über N-, O-, P- oder S-Atome) und/oder metallorganische Komplexe (d.h. Konjugation über das Metallatom) befinden. Analoges gilt für konjugierte Dendrimere.

Auch teilkonjugierte Polymere sind Gegenstand dieser Erfindung. Der Begriff "teilkonjugiertes Polymer" im Sinne dieser Erfindung bezeichnet Polymere, die konjugierte Regionen enthalten, die durch nicht konjugierte Abschnitte, gezielte Konjugationsunterbrecher (z.B. Abstandsgruppen, Sp) oder Verzweigungen voneinander getrennt sind. Konjugierte und teilkonjugierte Polymere können auch konjugierte, teilkonjugierte oder sonstige Dendrimere enthalten.

Unter dem Begriff "Dendrimer" soll hier eine hochverzweigte Verbindung verstanden werden, die aus einem multifunktionellen Zentrum (core) aufgebaut ist, an das in einem regelmäßigen Aufbau verzweigte Monomere gebunden werden, so dass eine baumartige Struktur erhalten wird. Dabei können sowohl das Zentrum als auch die Monomere beliebige verzweigte Strukturen annehmen, die sowohl aus rein organischen Einheiten als auch Organometallverbindungen oder Koordinationsverbindungen bestehen. "Dendrimer" soll hier allgemein so verstanden werden, wie dies beispielsweise von M. Fischer und F. Vögtle (Angew. Chem., Int. Ed. 1999, 38, 885) beschrieben ist.

Aryl(oxy)- und Heteroaryl(oxy)reste sind vorzugsweise ein- oder mehrfach mit L wie oben definiert substituiert.

Eine bevorzugte Ausführungsform der Erfindung richtet sich auf Verbindungen der Formel I, worin mindestens zwei der Reste R¹ bis R¹⁰, besonders bevorzugt die Reste R⁹ und R¹⁰, eine Abgangsgruppe X¹ wie oben definiert bedeuten. Solche Verbindungen eignen sich als Monomere zur Herstellung erfindungsgemäßer konjugierter Polymere und Dendrimere. Dabei kann ein Monomer der Formel I zu Homopolymeren umgesetzt werden oder mit anderen Monomeren der Formel I oder weiteren Monomeren zu Copolymeren umgesetzt werden. Vorzugsweise werden dabei ausschließlich solche Monomere verwendet, die zu vollständig konjugierten Polymeren führen.

Eine weitere bevorzugte Ausführungsform der Erfindung richtet sich auf Verbindungen der Formel I, worin einer oder mehrere Reste R¹ bis R¹⁰ oder R eine Gruppe P-Sp- wie oben definiert bedeuten oder eine Gruppe bedeuten, die mindestens einen Substituenten P-Sp- aufweist. Durch Polymerisation oder polymeranaloge Umsetzung der Gruppe P können aus solchen Verbindungen nicht-konjugierte, lineare oder vernetzte Polymere hergestellt werden, welche in ihrer Polymerhauptkette oder -seitenkette Strukturen der Formel I aufweisen. Entsprechend können aus Monomeren, die sowohl Abgangsgruppen X¹ als auch reaktive Gruppen P enthalten, zunächst durch Reaktion der Gruppen X¹ konjugierte Oligo- oder Polymere hergestellt werden, welche dann durch Reaktion der Gruppe P vernetzt werden.

Besonders bevorzugt sind Verbindungen der Formel I, worin einer, zwei, drei oder vier der Reste R¹⁻⁴ eine optional substituierte Arylgruppe mit 5 bis 40 C-Atomen bedeuten und die übrigen Reste R¹⁻⁴ H bedeuten.

Weiterhin bevorzugt sind Verbindungen der Formel I, worin
- alle Reste R¹⁻⁴ von H verschieden sind,
- R¹ von H verschieden ist und R²⁻⁴ H bedeuten,
- R¹ und R² von H verschieden sind und R³ und R⁴ H bedeuten
- R¹ und R⁴ von H verschieden sind und R² und R³ H bedeuten,
- alle von H verschiedenen Reste R¹⁻⁴ eine optional substituierte Arylgruppe bedeuten,
- die von H verschiedenen Reste R¹⁻⁴ Phenyl oder 1-Naphthyl bedeuten, welche optional mit ein oder mehreren, vorzugsweise 1, 2 oder 3 Resten L substituiert sind; ein Phenylrest ist vorzugsweise in 2-und/oder 5-Position mit L substituiert, ein 1-Naphthylrest ist vorzugsweise in 4-Position mit L substituiert,
- R⁹ und R¹⁰ jeweils unabhängig voneinander eine der für X¹ angegebenen Bedeutungen besitzen.

Ganz besonders bevorzugt sind Verbindungen der Formel I ausgewählt aus folgenden Unterformeln 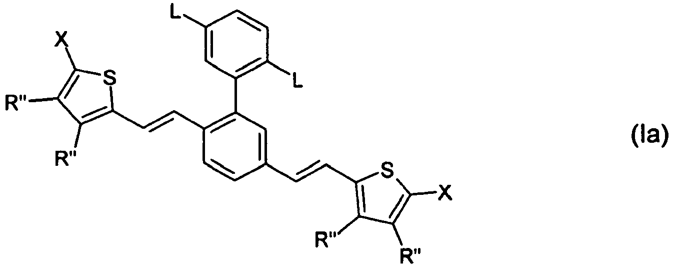 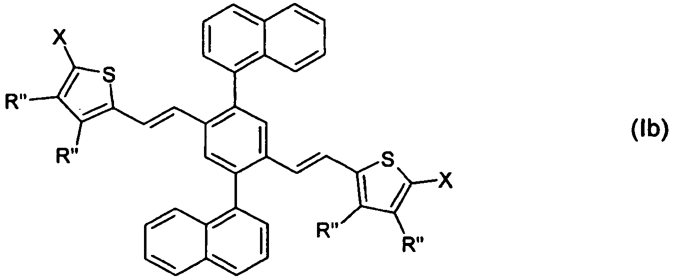 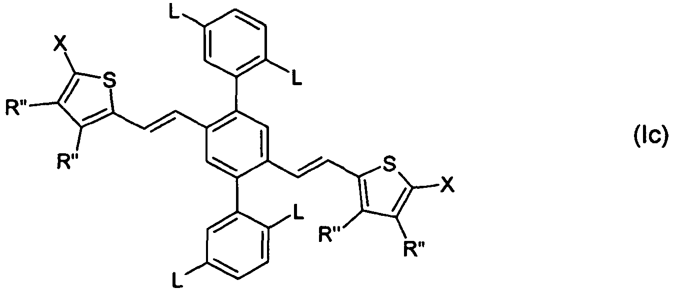 worin X eine der für X¹ angegebenen Bedeutungen besitzt, R" eine der für R⁵⁻⁸ angegebenen Bedeutungen besitzt und L eine der oben angegebenen Bedeutungen besitzt. L bedeutet in diesen Unterformeln vorzugsweise geradkettiges oder verzweigtes Alkyl oder Fluoroalkyl mit 1 bis 22, besonders bevorzugt 1 bis 12 C-Atomen.

Die Verbindungen der Formel I sind gut und in hohen Ausbeuten zugänglich. Sie zeigen im Feststoff intensive grüne Lumineszenz.

Die Verbindungen der Formel I können nach dem Fachmann bekannten und in der Literatur beschriebenen Methoden hergestellt werden. Weitere geeignete und bevorzugte Syntheseverfahren finden sich in den Beispielen. Die dort beschriebenen Verfahren sind ein weiterer Gegenstand der vorliegenden Erfindung.

Die erfindungsgemäßen Verbindungen und daraus hergestellte Polymere und Dendrimere weisen gegenüber den Materialien gemäß dem Stand der Technik verbesserte Eigenschaften, insbesondere deutlich verbesserte Lebensdauern, hohe Effizienzen und bessere Farbkoordinaten auf.

Die Verbindungen der Formel I können erfindungsgemäß in die Haupt-oder in die Seitenkette eines Polymers eingebaut werden. Bei Einbau in die Seitenkette besteht die Möglichkeit, dass die Verbindung der Formel I in Konjugation mit der Polymerhauptkette steht oder dass sie nicht-konjugiert zur Polymerhauptkette ist.

Falls die Verbindungen der Formel I zur Synthese von erfindungsgemäßen Polymeren verwendet werden, erfolgt die Verknüpfung im Polymer vorzugsweise über die Gruppen R⁹ und R¹⁰. Vorzugsweise werden für solche Verknüpfungsreaktionen Monomere der Formel I verwendet, worin R⁹ und R¹⁰ eine der für X¹ angegebenen Bedeutungen besitzen. Die Gruppen R^{9/10} bzw. X¹ werden bei dieser Verknüpfung eliminiert.

In der nachfolgenden Beschreibung der erfindungsgemäßen Polymere werden die Verbindungen der Formel I auch als "Struktureinheiten der Formel I" oder "Einheiten der Formel I" bezeichnet, wobei R⁹ und R¹⁰, bzw. bei Verknüpfung über andere Positionen analog die Reste R¹ bis R⁸, eine Verknüpfung im Polymer bedeuten sollen.

In einer bevorzugten Ausführungsform der Erfindung stehen Einheiten der Formel I in Konjugation mit der Polymerhauptkette. Dies kann einerseits dadurch erreicht werden, dass diese Einheiten in die Hauptkette des Polymers so eingebaut werden, dass dadurch die Konjugation des Polymers, wie oben beschrieben, erhalten bleibt. Andererseits können diese Einheiten auch in die Seitenkette des Polymers so verknüpft werden, dass eine Konjugation zur Hauptkette des Polymers besteht. Dies ist beispielsweise der Fall, wenn die Verknüpfung mit der Hauptkette nur über sp²-hybridisierte (bzw. gegebenenfalls auch über sp-hybridisierte) Kohlenstoffatome, die auch durch entsprechende Heteroatome ersetzt sein können, erfolgt. Erfolgt die Verknüpfung jedoch durch Einheiten, wie beispielsweise einfache (Thio)Etherbrücken, Ester, Amide oder Alkylenketten, so sind die Einheiten der Formel I als nicht-konjugiert zur Hauptkette definiert.

Erfindungsgemäße konjugierte oder teilkonjugierte Polymere und Dendrimere enthalten vorzugsweise von 0,01 bis 25 mol% einer oder mehrerer Einheiten der Formel I. Die einzusetzende Menge hängt von der zu erzielenden Farbe des Polymers ab. Bevorzugt für einfarbige Polymere sind 0,8 bis 20, besonders bevorzugt 1 bis 15 mol%. Für mehrfarbige Polymere sind die Anteile geringer, vorzugsweise unter 1,5 mol%, besonders bevorzugt unter 1 mol%.

Besonders bevorzugt sind erfindungsgemäße Polymere, die neben Einheiten gemäß Formel I noch weitere Strukturelemente enthalten, und somit als Copolymere zu bezeichnen sind. Die weiteren Comonomere sind zwar notwendig zur Synthese der erfindungsgemäßen Copolymere; sie sind allerdings nicht selbst Gegenstand der vorliegenden Erfindung und sind somit durch Zitat zu beschreiben. Hier sei vor allem auch auf die relativ umfangreichen Auflistungen in der WO 02/077060, der WO 2005/014689 und der darin aufgeführten Zitate verwiesen. Diese weiteren Struktureinheiten können beispielsweise aus den im Folgenden beschriebenen Klassen stammen:
- Gruppe 1:: Comonomere, welche das Polymer-Grundgerüst darstellen.
- Gruppe 2:: Comonomere, welche die Lochinjektions- und/oder -transporteigenschaften der Polymere erhöhen.
- Gruppe 3:: Comonomere, welche die Elektroneninjektions- und/oder -transporteigenschaften der Polymere erhöhen.
- Gruppe 4:: Comonomere, die Kombinationen von Einzeleinheiten der Gruppe 2 und Gruppe 3 aufweisen.

Geeignete und bevorzugte Einheiten für die oben genannten Gruppen werden im Folgenden beschrieben.

Gruppe 1 - Comonomere, welche das Polymer-Grundgerüst darstellen: Bevorzugte Einheiten der Gruppe 1 sind insbesondere solche, die aromatische oder carbocyclische Strukturen mit 6 bis 40 C-Atomen beinhalten. Geeignete und bevorzugte Einheiten sind unter anderem Fluoren-Derivate wie z. B. in EP 0 842 208, WO 99/54385, WO 00/22027, WO 00/22026 oder WO 00/46321 offenbart, ferner Spirobifluoren-Derivate wie z.B. in EP 0 707 020, EP 0 894 107 und WO 03/020790 offenbart, oder Dihydrophenanthren-Derivate wie in WO 2005/014689 offenbart. Es ist auch möglich, eine Kombination von zwei oder mehr dieser MonomerEinheiten zu verwenden, wie z.B. in WO 02/077060 beschrieben. Es sind auch andere Strukturelemente möglich, die die Morphologie, aber auch die Emissionsfarbe der resultierenden Polymere beeinflussen können. Bevorzugt sind dabei substituierte oder unsubstituierte aromatische Strukturen, die 6 bis 40 C-Atome aufweisen oder auch Stilben- oder Bisstyrylarylenderivate, wie z.B. 1,4-Phenylen-, 1,4-Naphthylen-, 1,4- oder 9,10-Anthrylen-, 1,6- oder 2,7- oder 4,9-Pyrenylen-, Tetrahydropyrenylen-, 3,9- oder 3,10-Perylenylen-, 2,7- oder 3,6-Phenanthrenylen-, 4,4'-Biphenylylen-, 4,4"-Terphenylylen-, 4,4'-Bi-1,1'-naphthylylen-, 4,4'-Stilbenyl- oder 4,4"-Bisstyrylarylenderivaten.

Bevorzugte Einheiten für das Polymergrundgerüst sind Spirobifluorene, Indenofluorene, Phenanthrene und Dihydrophenanthrene.

Besonders bevorzugte Einheiten der Gruppe 1 sind zweibindige Einheiten gemäß den folgenden Formeln, worin die gestrichelte Linie die Verknüpfung zur benachbarten Einheit bedeutet: 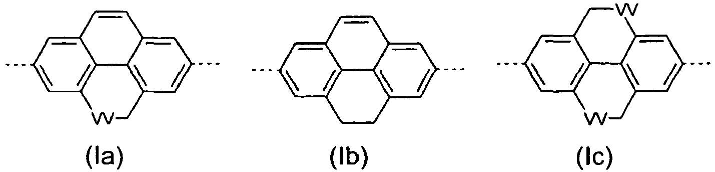 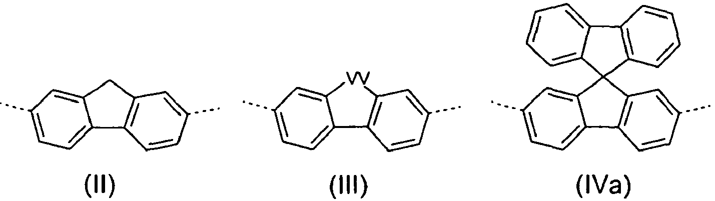 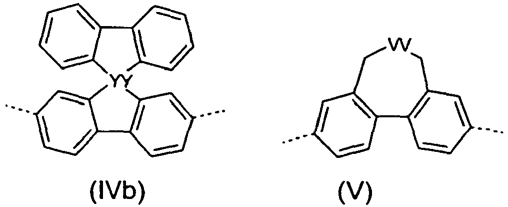 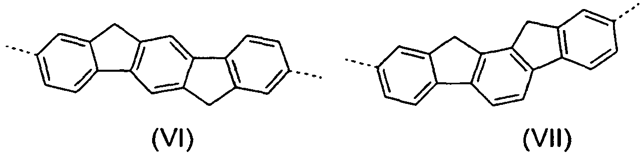 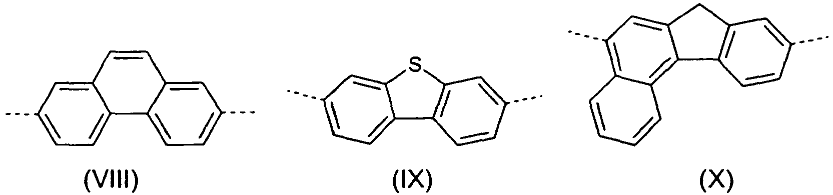 wobei die verschiedenen Positionen auch durch einen oder mehrere Substituenten R⁵ wie oben definiert substituiert sein können, YY Si oder Ge bedeutet und W O, S oder Se bedeutet.

Gruppe 2 - Comonomere, welche die Lochinjektions- und/oder -transporteigenschaften der Polymere erhöhen:

Dies sind im Allgemeinen aromatische Amine oder elektronenreiche Heterocyclen, wie beispielsweise substituierte oder unsubstituierte Triarylamine, Benzidine, Tetraarylen-para-phenylendiamine, Phenothiazine, Phenoxazine, Dihydrophenazine, Thianthrene, Dibenzo-p-dioxine, Phenoxathiine, Carbazole, Azulene, Thiophene, Pyrrole, Furane und weitere O-, S- oder N-haltige Heterocyclen mit hoch liegendem HOMO (HOMO = höchst liegendes besetztes Molekülorbital). Es kommen hier aber auch Triarylphosphine in Frage, wie in WO 2005/017065 beschrieben.

Besonders bevorzugte Einheiten der Gruppe 2 sind zweibindige Einheiten gemäß den folgenden Formeln, worin die gestrichelte Linie die Verknüpfung zur benachbarten Einheit bedeutet: 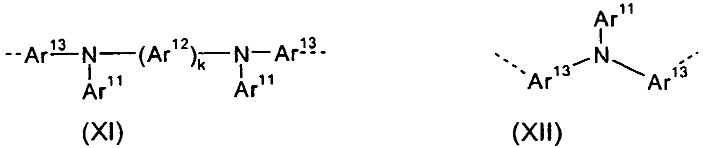 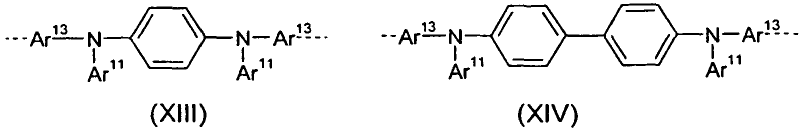 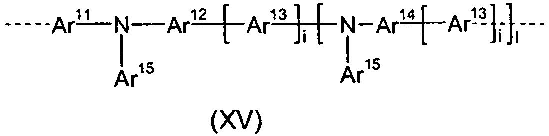 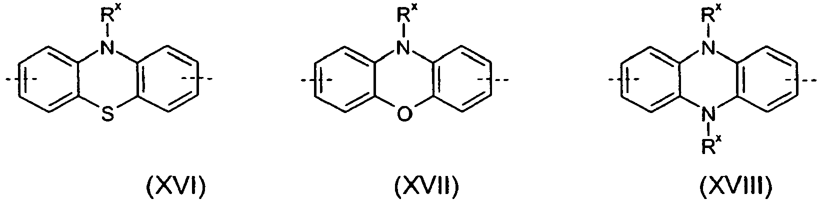 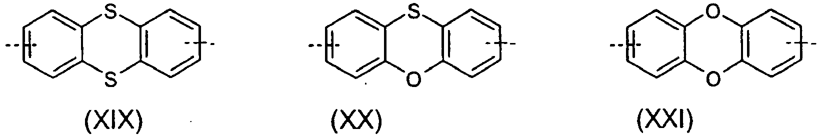 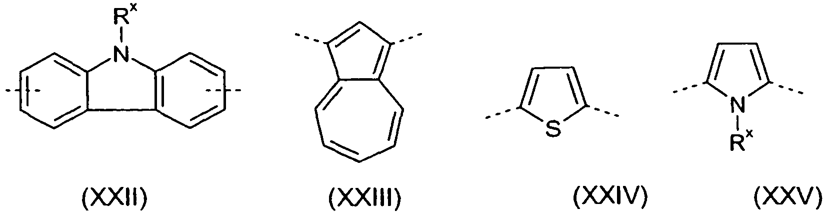 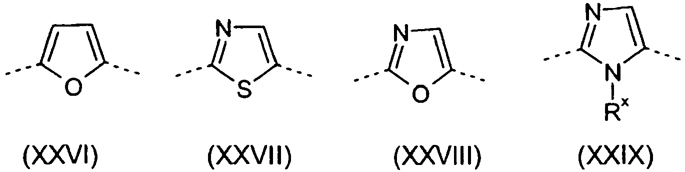 wobei R^{x} eine der oben für R¹ angegebenen Bedeutungen besitzt, die verschiedenen Formeln an den freien Positionen auch zusätzlich durch einen oder mehrere Substituenten R^{x} substituiert sein können und die weiteren Symbole und Indices folgendes bedeuten:
- i: ist gleich oder verschieden bei jedem Auftreten 0, 1 oder 2,
- k: ist gleich oder verschieden bei jedem Auftreten 0, 1 oder 2, bevorzugt 0 oder 1,
- l: ist gleich oder verschieden bei jedem Auftreten 1, 2 oder 3, bevorzugt 1 oder 2,
- Ar¹¹, Ar¹³: sind bei jedem Auftreten gleich oder verschieden ein aromatisches oder heteroaromatisches Ringsystem mit 2 bis 40 C-Atomen, welches durch R^{x} ein- oder mehrfach substituiert oder auch unsubstituiert sein kann; die möglichen Substituenten R^{x} können dabei potentiell an jeder freien Position sitzen,
- Ar¹², Ar¹⁴: sind bei jedem Auftreten gleich oder verschieden Ar¹¹, Ar¹³ oder eine substituierte oder unsubstituierte Stilbenylen- bzw. Tolanyleneinheit,
- Ar¹⁵: ist gleich oder verschieden bei jedem Auftreten entweder ein System gemäß Ar¹¹ oder ein aromatisches oder heteroaromatisches Ringsystem mit 9 bis 40 aromatischen Atomen (C- oder Heteroatomen), welches durch R^{x} ein- oder mehrfach substituiert oder unsubstituiert sein kann und welches aus mindestens zwei kondensierten Ringen besteht; die möglichen Substituenten R^{x} können dabei potentiell an jeder freien Position sitzen.

Gruppe 3 - Comonomere, welche die Elektroneninjektions- und/oder -transporteigenschaften der Polymere deutlich erhöhen:

Dies sind im Allgemeinen elektronenarme Aromaten oder Heterocyclen, wie beispielsweise substituierte oder unsubstituierte Pyridine, Pyrimidine, Pyridazine, Pyrazine, Anthracene, Oxadiazole, Chinoline, Chinoxaline oder Phenazine, aber auch Verbindungen wie Triarylborane und weitere O-, S- oder N-haltige Heterocyclen mit niedrig liegendem LUMO (LUMO = niedrigstes unbesetztes Molekülorbital), sowie Benzophenone und deren Derivate, wie z.B. in WO 05/040302 offenbart.

Besonders bevorzugte Einheiten der Gruppe 3 sind zweibindige Einheiten gemäß den folgenden Formeln, worin die gestrichelte Linie die Verknüpfung zur benachbarten Einheit bedeutet: 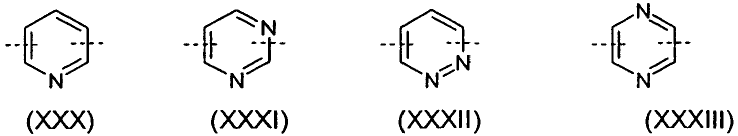 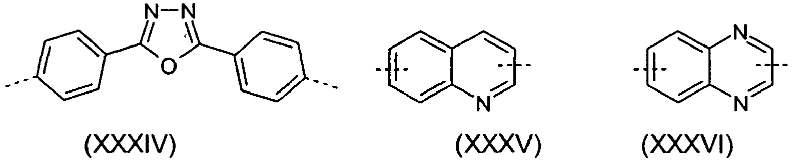 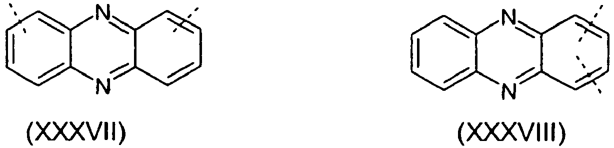 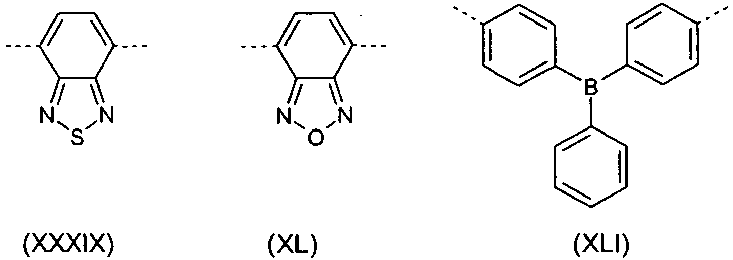 wobei die verschiedenen Formeln an den freien Positionen durch einen oder mehrere Substituenten R^{x} wie oben definiert substituiert sein können.

Gruppe 4 - Comonomere, die Kombinationen von Einzeleinheiten der Gruppe 2 und Gruppe 3 aufweisen:

Es ist auch möglich, dass die erfindungsgemäßen Polymere Einheiten enthalten, in denen Strukturen, welche die Lochmobilität und welche die Elektronenmobilität erhöhen, direkt aneinander gebunden sind. Allerdings verschieben einige dieser Einheiten die Emissionsfarbe ins Gelbe oder Rote. Ihre Verwendung in erfindungsgemäßen Polymeren zur Erzeugung blauer oder grüner Emission ist deshalb weniger bevorzugt.

Falls solche Einheiten der Gruppe 4 in den erfindungsgemäßen Polymeren enthalten sind, sind sie bevorzugt ausgewählt aus zweibindigen Einheiten gemäß den folgenden Formeln, worin die gestrichelte Linie die Verknüpfung zur benachbarten Einheit bedeutet: 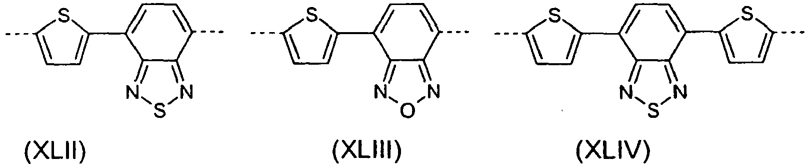 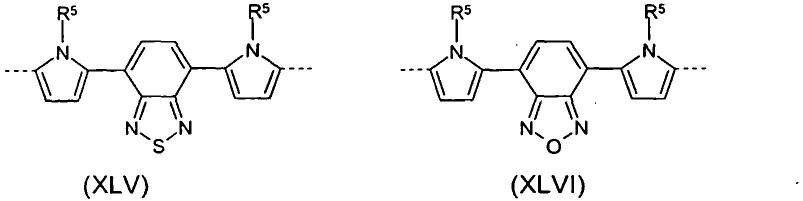 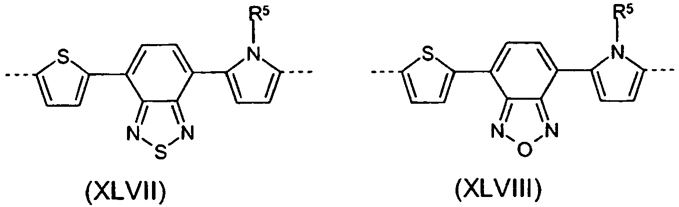 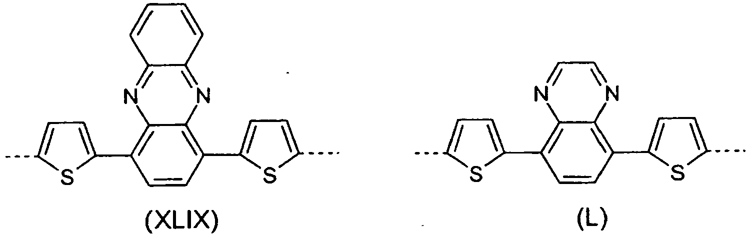 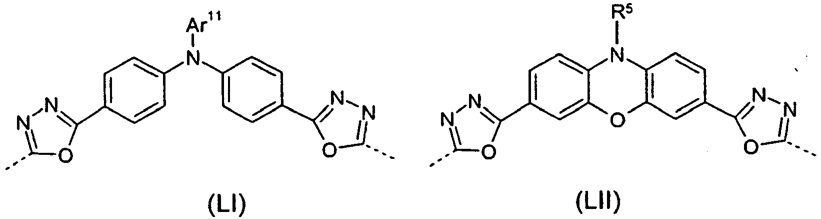 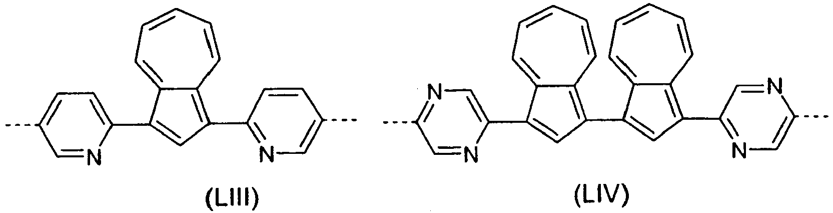 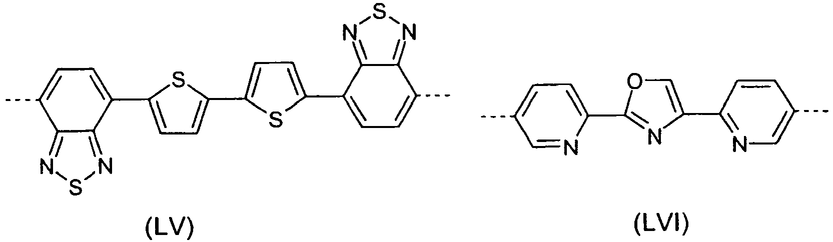 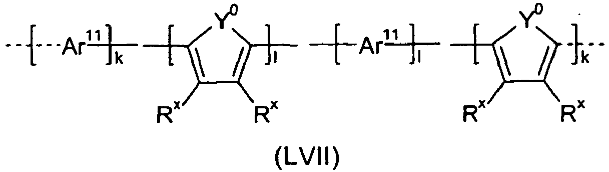 wobei die verschiedenen Formeln an den freien Positionen durch einen oder mehrere Substituenten R^{x} wie oben definiert substituiert sein können, die Symbole R^{x}, Ar¹¹, k und I die oben genannte Bedeutung besitzen und Y⁰ bei jedem Auftreten gleich oder verschieden O, S, Se, N, P, Si oder Ge ist.

Es ist auch möglich, dass gleichzeitig mehr als eine Struktureinheit aus einer der Gruppen 1 bis 4 vorliegt.

Das erfindungsgemäße Polymer kann weiterhin ebenfalls in die Haupt-oder Seitenkette gebundene Metallkomplexe enthalten, die im Allgemeinen aus einem oder mehreren Liganden und einem oder mehreren Metallzentren aufgebaut sind.

Bevorzugt sind erfindungsgemäße Polymere, die neben Einheiten gemäß Formel I zusätzlich noch eine oder mehrere Einheiten ausgewählt aus den Gruppen 1 bis 4 enthalten.

Bevorzugt sind dabei erfindungsgemäße Polymere, die neben Einheiten gemäß Formel I noch Einheiten aus der Gruppe 1 enthalten, besonders bevorzugt mindestens 50 mol% dieser Einheiten.

Ebenfalls bevorzugt ist es, wenn die erfindungsgemäßen Polymere Einheiten enthalten, die den Ladungstransport oder die Ladungsinjektion verbessern, also Einheiten aus Gruppe 2 und/oder 3; besonders bevorzugt ist ein Anteil von 2 bis 30 mol% dieser Einheiten; ganz besonders bevorzugt ist ein Anteil von 2 bis 10 mol% dieser Einheiten.

Besonders bevorzugt ist es weiterhin, wenn die erfindungsgemäßen Polymere Einheiten aus Gruppe 1 und Einheiten aus Gruppe 2 und/oder 3 enthalten, insbesondere mindestens 50 mol% Einheiten aus Gruppe 1 und 2 bis 30 mol% Einheiten aus Gruppe 2 und/oder 3.

Die erfindungsgemäßen Polymere weisen vorzugsweise 10 bis 10000, besonders bevorzugt 20 bis 5000 und insbesondere 50 bis 2000 Wiederholeinheiten auf. Entsprechende Dendrimere können auch weniger Wiederholeinheiten aufweisen.

Die nötige Löslichkeit der Polymere und Dendrimere wird vor allem durch die Substituenten an den verschiedenen Wiederholeinheiten gewährleistet, sowohl den Substituenten R¹⁻¹⁰ an den Verbindungen gemäß Formel I, wie auch durch Substituenten an den anderen Wiederholeinheiten.

Die erfindungsgemäßen Polymere sind entweder Homopolymere aus Einheiten der Formel I oder Copolymere. Die erfindungsgemäßen Polymere können linear oder verzweigt (vernetzt) sein. Erfindungsgemäße Copolymere können dabei neben einer oder mehreren Einheiten der Formel I, oder deren bevorzugten Unterformeln, potentiell eine oder mehrere weitere Strukturen aus den oben aufgeführten Gruppen 1 bis 4 besitzen.

Die erfindungsgemäßen Copolymere können statistische, alternierende oder blockartige Strukturen aufweisen oder auch mehrere dieser Strukturen abwechselnd besitzen. Wie Copolymere mit blockartigen Strukturen erhalten werden können und welche weiteren Strukturelemente dafür besonders bevorzugt sind, ist beispielsweise ausführlich in WO 2005/014688 beschrieben. Diese ist via Zitat Bestandteil der vorliegenden Anmeldung. Ebenso sei an dieser Stelle nochmals hervorgehoben, dass das Polymer auch dendritische Strukturen haben kann.

Verbindungen der Formel I können auch als grün emittierende Comonomere für die Synthese rot emittierender Polymere verwendet werden. Weiterer Gegenstand der Erfindung ist also die Verwendung von Verbindungen der Formel I für die Synthese rot emittierender Polymere.

Es kann auch bevorzugt sein, dass ein deutlich geringerer Anteil als 1 mol% Verbindungen der Formel I verwendet wird. So können 0,01 bis 1 mol% solcher Verbindungen zum Beispiel als blau oder grün emittierende Einheiten für die Synthese weiß emittierender Copolymere verwendet werden. Hierfür wird im Allgemeinen nur ein geringer Anteil blau bzw. grün emittierender Einheiten benötigt, wie in WO 2005/030828 beschrieben. Gegenstand der Erfindung ist also auch die Verwendung von Verbindungen der Formel I für die Synthese weiß emittierender Copolymere.

Vorzugsweise enthalten erfindungsgemäße, weiß emittierende Copolymere mindestens drei unterschiedliche Wiederholeinheiten, wobei die erste Wiederholeinheit, Einheit B, üblicherweise in einem Anteil von mindestens 10 mol% vorliegt und blaue Emission zeigt, die zweite Wiederholeinheit, Einheit G, üblicherweise in einem Anteil von 0,001 bis 3 mol% im Polymer vorliegt und grüne Emission zeigt und die dritte Wiederholeinheit, Einheit **R**, üblicherweise in einem Anteil von 0,0005 bis 1 mol% vorliegt und rote Emission zeigt. Die grüne Wiederholeinheit ist dabei eine Einheit der Formel I. Es können jedoch auch mehrere Einheiten **R**, G und B vorliegen, wobei jedoch mindestens eine der Einheiten G der Formel I entspricht.

Weiße Emission ist definiert durch die CIE-Farbkoordinaten x = 0,33 und y = 0,33 (Chromatizitäts-Koordinaten der Commission Internationale de l'Eclairage von 1931). Der Farbeindruck kann jedoch individuell verschieden sein, so dass auch ein Wert, der in der Nähe dieses Bereiches liegt, immer noch den Eindruck weißer Emission hinterlassen kann. Unter weißer Emission im Sinne dieser Erfindung soll eine Emission verstanden werden, deren Farbkoordinaten innerhalb einer Ellipse liegen, die von den Punkten mit den x / y-Farbkoordinaten von ca. (0,22 / 0,24), (0,46 / 0,44), (0,28 / 0,38) und (0,37 / 0,28) aufgespannt wird.

Eine blau emittierende Wiederholeinheit B im Sinne dieser Anmeldung ist so definiert, dass ein Film des Homopolymers dieser Einheit B Lumineszenz (Fluoreszenz oder Phosphoreszenz) zeigt und dass das Maximum der Emissionskurve eines Films eines Polymers, das 10 mol% dieser Einheit B und 90 mol% 2,7-[2',3',6',7'-tetra(2-methylbutyloxy)spirobifluoren]ylen enthält, in einem Wellenlängenbereich von 400 bis 490 nm liegt.

Eine grün emittierende Wiederholeinheit G im Sinne dieser Anmeldung ist so definiert, dass das Maximum der Fluoreszenz- oder Phosphoreszenzkurve eines Films eines Polymers, das 10 mol% dieser Einheit G und 90 mol% 2,7-[2',3',6',7'-tetra(2-methylbutyloxy)spirobifluoren]ylen enthält, in einem Wellenlängenbereich von 490 bis 570 nm liegt.

Eine rot emittierende Wiederholeinheit R im Sinne dieser Anmeldung ist so definiert, dass das Maximum der Fluoreszenz- oder Phosphoreszenzkurve eines Films eines Polymers, das 10 mol% dieser Einheit R und 90 mol% 2,7-[2',3',6',7'-tetra(2-methylbutyloxy)spirobifluoren]ylen enthält, in einem Wellenlängenbereich von 570 bis 700 nm liegt.

Es sei hier ausdrücklich darauf hingewiesen, dass auch Mischfarben, wie beispielsweise gelb oder orange im Sinne dieser Erfindung je nach ihrem Emissionsmaximum zu roter oder grüner Emission zugerechnet werden sollen.

Als blau emittierende Wiederholeinheiten B kommen typischerweise Einheiten in Frage, welche im Allgemeinen als Polymergrundgerüst ("Backbone") verwendet werden oder solche, welche als blaue Emitter verwendet werden. Dies sind im Allgemeinen solche, welche mindestens eine aromatische oder andere konjugierte Struktur aufweisen, aber die Emissionsfarbe nicht ins Grüne oder ins Rote verschieben. Bevorzugt sind aromatische Strukturen mit 4 bis 40 C-Atomen, aber auch Stilben- und Tolan-Derivate und gewisse Bis(styryl)arylen-Derivate. Dies wären beispielsweise folgende Strukturelemente, die substituiert oder unsubstituiert sein können: 1,4-Phenylen-, 1,4-Naphthylen-, 1,4- oder 9,10-Anthracenylen-, 2,7- oder 3,6-Phenanthrenylen-, 4,4'-Biphenylylen-, 4,4"-Terphenylylen-, 4,4'-Bi-1,1'-naphthylylen-, 4,4'-Stilbenderivate, 4,5-Dihydropyrenderivate, 4,5,9,10-Tetrahydropyrenderivate (z.B. gemäß EP 0 699 699), Fluorenderivate (z.B. gemäß EP 0 842 208, WO 99/54385, WO 00/22027, WO 00/22026, WO 00/46321), Spirobifluorenderivate (z.B. gemäß EP 0 707 020, EP 0 894 107, WO 03/020790, WO 02/077060), 5,7-Dihydrodibenzoxepinderivate, cis- und trans-Indenofluorenderivate (z.B. gemäß WO 2004/041901 und WO 2004/113412) und 9,10-Dihydrophenanthrenderivate (z.B. gemäß WO 2005/014689). Neben diesen Klassen kommen hier beispielsweise auch die sogenannten Leiter-PPPs ("Ladder-PPPs" = LPPP) (z.B. gemäß WO 92/18552), aber auch Ansa-Strukturen enthaltende PPPs (z.B. gemäß EP 0 690 086) in Frage. Auch Bis(styryl)arylen-Derivate, die nicht elektronenreich sind, können hierfür verwendet werden.

Es kann auch bevorzugt sein, wenn mehr als eine solche blau emittierende Wiederholeinheit B in einem Polymer verwendet wird.

Falls das Polymer zusätzlich zu den Einheiten der Formel I weitere grün emittierende Wiederholeinheiten G enthält, kommen hierfür bevorzugt Einheiten in Frage, welche mindestens eine aromatische oder sonstige konjugierte Struktur aufweisen und die Emissionsfarbe ins Grüne verschieben. Bevorzugte Strukturen für grün emittierende Wiederholeinheiten G sind ausgewählt aus den Gruppen der elektronenreichen Bisstyrylarylene und Derivate dieser Strukturen. Ohne dabei an eine bestimmte Theorie gebunden sein zu wollen, führt eine elektronenschiebende Substitution zu einer Grünverschiebung der Emission. Weitere bevorzugte grün emittierende Wiederholeinheiten sind ausgewählt aus den Gruppen der Benzothiadiazole und entsprechender Sauerstoffderivate, der Chinoxaline, der Phenothiazine, der Phenoxazine, der Dihydrophenazine, der Bis(thiophenyl)arylene, der Oligo(thiophenylene) und der Phenazine. Dabei ist es auch zulässig, dass statt einer grün emittierenden Wiederholeinheit G mehrere verschiedene solcher Wiederholeinheiten verwendet werden, wobei dann der Gesamtanteil der grün emittierenden Wiederholeinheiten G maximal 3 mol% beträgt.

Besonders bevorzugte Strukturen, die sich als grün emittierende Wiederholeinheiten G eignen, sind Strukturen gemäß der folgenden Formeln 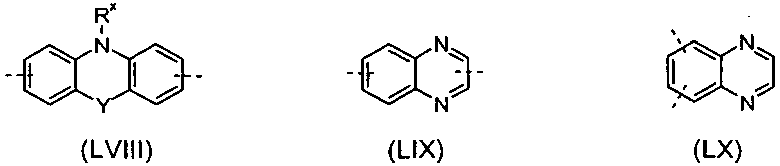 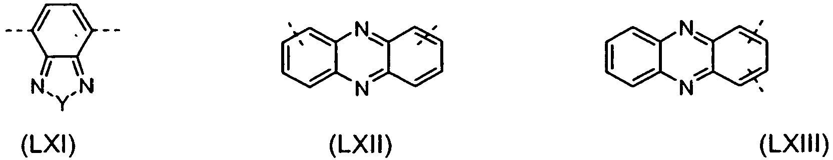 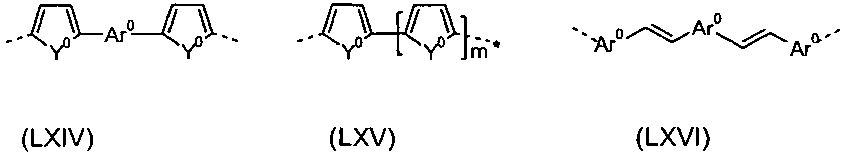 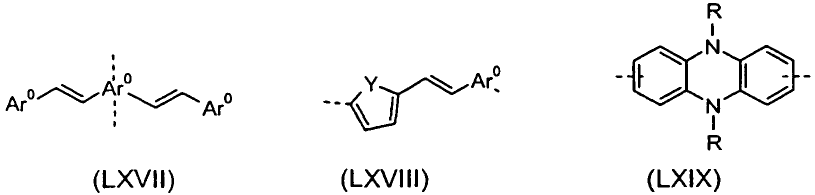 welche substituiert oder unsubstituiert sein können, wobei für die verwendeten Symbole und Indices Folgendes gilt:
Y⁰ ist vorzugsweise bei jedem Auftreten gleich oder verschieden S oder O;
Ar⁰ ist bei jedem Auftreten gleich oder verschieden eine Arylengruppe, ausgewählt aus den Gruppen der Phenylene, Biphenylene, Fluorenylene, Spirobifluorenylene, Thienylene, Furanylene, Pyrrolylene, mit der Vorgabe, dass in Formel (LXVI) und (LXVII) mindestens eine Ar⁰-Einheit eine elektronenreiche aromatische Einheit sein muss; dies wird dadurch erreicht, dass diese Einheit ausgesucht ist aus den Strukturen der substituierten oder unsubstituierten Thionylene, Furanylene oder Pyrrolylene oder dass diese Einheit eine Phenylengruppe ist, die mit mindestens einer Alkoxy-, Aryloxy- oder substituierten oder unsubstituierten Aminogruppe oder auch mehrerer gleicher oder verschiedener solcher Gruppen substituiert ist;
R^{x} ist vorzugsweise bei jedem Auftreten gleich oder verschieden H, eine geradkettige, verzweigte oder cyclische Alkylkette mit 1 bis 22 C-Atomen, in der auch ein oder mehrere nicht benachbarte C-Atome durch O, S, -CO-O- oder -O-CO-O- ersetzt sein können, wobei auch ein oder mehrere H-Atome durch Fluor ersetzt sein können, eine substituierte oder unsubstituierte Arylgruppe mit 5 bis 40 C-Atomen, bei der auch ein oder mehrere C-Atome durch O, S oder N ersetzt sein können;
m ist bei jedem Auftreten gleich oder verschieden 1, 2, 3, 4 oder 5, vorzugsweise 1, 2 oder 3;
dabei sollen die gestrichelten Bindungen die Verknüpfung im Polymer andeuten; sie stehen hier nicht für eine Methylgruppe.

Als rot emittierende Wiederholeinheiten R kommen vorzugsweise Einheiten in Frage, welche mindestens eine aromatische oder sonstige konjugierte Struktur aufweisen und die Emissionsfarbe ins Rote verschieben. Bevorzugte Strukturen für rot emittierende Wiederholeinheiten R sind solche, in denen elektronenreiche Einheiten, wie z.B. Thiophen, mit grün emittierenden elektronenarmen Einheiten, wie z.B. Chinoxalin oder Benzothiadiazol, kombiniert sind. Weitere bevorzugte rot emittierende Wiederholeinheiten R sind Systeme aus mindestens vier kondensierten aromatischen Einheiten, wie z.B. Rubrene, Pentacene oder Perylene, die vorzugsweise substituiert sind, oder vorzugsweise konjugierte Push-Pull-Systeme (Systeme, die mit Donor- und Akzeptorsubstituenten substituiert sind) oder Systeme wie Squarine oder Chinacridone, die vorzugsweise substituiert sind. Dabei ist es auch zulässig, dass statt einer rot emittierenden Wiederholeinheit R mehrere solcher Wiederholeinheiten verwendet werden, wobei dann der Gesamtanteil der rot emittierenden Wiederholeinheiten R maximal 1 mol% beträgt.

Besonders bevorzugte Strukturen, die sich als rot emittierende Wiederholeinheiten R eignen, sind Strukturen gemäß den folgenden Formeln, 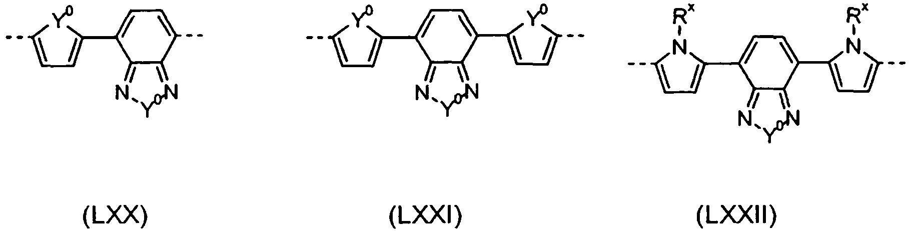 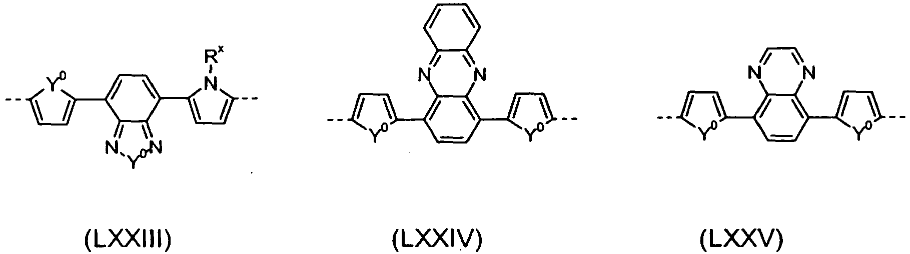 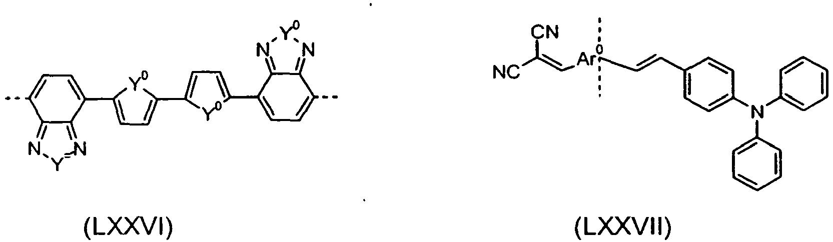 welche substituiert oder unsubstituiert sein können, wobei die Symbole und Indices dieselbe Bedeutung haben wie oben beschrieben.

Als blau, grün und rot emittierende Struktureinheiten B, G und R kommen prinzipiell auch Einheiten in Frage, die aus dem Triplett-Zustand Licht emittieren, also Elektrophosphoreszenz statt Elektrofluoreszenz zeigen, was häufig eine Steigerung der Energieeffizienz bewirkt. Diese Einheiten werden im Folgenden als Triplett-Emitter bezeichnet. Die Verwendung derartiger Metallkomplexe in niedermolekularen OLEDs ist beispielsweise in M. A. Baldo et al. (Appl. Phys. Lett. 1999, 75, 4-6) beschrieben. Hierfür eignen sich zunächst Verbindungen, welche Schweratome, d.h. Atome aus dem Periodensystem der Elemente mit einer Ordnungszahl von mehr als 36, enthalten. Besonders geeignet hierfür sind Verbindungen, welche d- und f-Übergangsmetalle beeinhalten, die die oben genannte Bedingung erfüllen. Ganz besonders bevorzugt sind hier entsprechende Struktureinheiten, welche Elemente der Gruppe 8 bis 10 (d.h. Ru, Os, Rh, Ir, Pd, Pt) enthalten. Als Struktureinheiten für die erfindungsgemäßen Polymeren kommen z.B. verschiedene Komplexe in Frage, welche beispielsweise in WO 02/068435, DE 101 16 962, EP 1 239 526 und WO 2004/026886 beschrieben sind. Entsprechende Monomere sind in der WO 02/068435 beschrieben.

Die Farben der Komplexe werden dabei in erster Linie vom verwendeten Metall, von der genauen Ligandenstruktur und von den Substituenten am Liganden bestimmt. Es sind sowohl grün als auch rot emittierende Komplexe bekannt. So emittiert beispielsweise ein unsubstituiertes Tris(phenyl-pyridyl)-iridium(III) grünes Licht, während elektronenschiebende Substituenten in para-Stellung zum koordinierenden Kohlenstoffatom (z.B. Diarylamino-Substituenten) die Emission ins Orange-Rote verschieben. Weiterhin sind Derivate dieses Komplexes mit variierter Ligandenstruktur bekannt, die direkt (ohne weitere Substitutionen) zu orange oder tiefroter Emission führen. Beispiele für solche Liganden sind 2-Phenylisochinolin, 2-Benzothiophenylpyridin oder 2-Naphthylpyridin.

Blau emittierende Komplexe werden beispielsweise erhalten, indem der Tris(phenyl-pyridyl)-iridium(III)-Grundkörper mit elektronenziehenden Substituenten wie beispielsweise mehreren Fluor- und/oder Cyanogruppen substituiert wird.

Die erfindungsgemäßen Polymere werden in der Regel durch Polymerisation von einer oder mehreren Monomersorten hergestellt, von denen mindestens ein Monomer durch die Formel I beschrieben ist. Geeignete Polymerisationsreaktionen sind dem Fachmann bekannt und in der Literatur beschrieben. Besonders geeignete und bevorzugte Polymerisationsreaktionen, die alle zu C-C-Verknüpfungen führen, sind folgende:
(A) Polymerisation gemäß SUZUKI;
(B) Polymerisation gemäß YAMAMOTO;
(C) Polymerisation gemäß STILLE;

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 03/048225 oder WO 2004/037887, im Detail beschrieben.

Die C-C-Verknüpfungen sind bevorzugt ausgewählt aus den Gruppen der SUZUKI-Kupplung, der YAMAMOTO-Kupplung und der STILLE-Kupplung.

Die erfindungsgemäßen Dendrimere können gemäß dem Fachmann bekannten Verfahren oder in Analogie dazu hergestellt werden. Geeignete Verfahren sind in der Literatur beschrieben, wie z.B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6, WO 02/067343 A1 oder WO 2005/026144 A1.

Zur Synthese der Polymere und Dendrimere werden die entsprechenden Monomere benötigt. Die Synthese der oben beschriebenen Einheiten aus Gruppe 1 bis 4 und des Typs **R**, **G** und **B** ist dem Fachmann bekannt und in der Literatur, beispielsweise in WO 2005/014689, WO 2005/030827 und WO 2005/030828, beschrieben. Diese und die darin zitierte Literatur ist via Zitat Bestandteil der vorliegenden Anmeldung.

Es kann außerdem bevorzugt sein, erfindungsgemäße Polymere oder Dendrimere nicht als Reinsubstanz, sondern als Blend (Mischung) zusammen mit weiteren beliebigen polymeren, oligomeren, dendritischen oder niedermolekularen Substanzen zu verwenden. Diese können z.B. die elektronischen Eigenschaften verbessern oder selber emittieren. Solche Blends sind daher auch Bestandteil der vorliegenden Erfindung.

Als "Blend" wird vor- und nachstehend eine Mischung enthaltend mindestens eine oligomere oder polymere Komponente bezeichnet.

Gegenstand der Erfindung sind weiterhin Lösungen und Formulierungen aus einem oder mehreren erfindungsgemäßen Verbindungen, Polymeren, Dendrimeren, Blends oder Mischungen in einem oder mehreren Lösungsmitteln. Wie solche Lösungen hergestellt werden können, ist dem Fachmann bekannt und beispielsweise in WO 02/072714, WO 03/019694 und der darin zitierten Literatur beschrieben.

Diese Lösungen können verwendet werden, um dünne Polymerschichten herzustellen, zum Beispiel durch Flächenbeschichtungsverfahren (z.B. Spin-coating) oder durch Druckverfahren (z.B. InkJet Printing).

Verbindungen der Formel I, Polymere oder Dendrimere enthaltend Struktureinheiten der Formel I, welche eine oder mehrere Gruppen X¹-Sp-wie oben beschrieben enthalten, eignen sich besonders zur Herstellung von Filmen oder Beschichtungen, insbesondere zur Herstellung von strukturierten Beschichtungen, z.B. durch thermische oder lichtinduzierte in-situ-Polymerisation und in-situ-Vernetzung, wie beispielsweise in-situ-UV-Photopolymerisation oder Photopatterning. Besonders bevorzugt für solche Anwendungen sind erfindungsgemäße Verbindungen, Polymere oder Dendrimere mit einer oder mehreren Gruppen X¹-Sp-, worin X¹ Acrylat, Methacrylat, Vinyl, Epoxy oder Oxetan bedeutet. Dabei können sowohl entsprechende Verbindungen der Formel I und deren Polymere in Reinsubstanz verwendet werden, es können aber auch Mischungen, Formulierungen oder Blends dieser Verbindungen/Polymere wie oben beschrieben verwendet werden. Diese können mit oder ohne Zusatz von Lösungsmitteln und/oder Bindemitteln verwendet werden. Geeignete Materialien, Verfahren und Vorrichtungen für die oben beschriebenen Methoden sind z.B. in WO 2005/083812 beschrieben. Mögliche Bindemittel sind beispielsweise Polystyrol, Polycarbonat, Polyacrylate, Polyvinylbutyral und ähnliche, optoelektronisch neutrale Polymere.

Geeignete und bevorzugte Lösungsmittel sind beispielsweise Toluol, Anisol, Xylol, Methylbenzoate, Dimethylanisol, Mesitylen, Tetralin, Veratrol und Tetrahydrofuran.

Die erfindungsgemäßen Polymere, Dendrimere, Blends und Formulierungen können in PLEDs verwendet werden. Wie PLEDs hergestellt werden können, ist dem Fachmann bekannt und wird beispielsweise als allgemeines Verfahren ausführlich in WO 2004/070772 beschrieben, das entsprechend für den Einzelfall anzupassen ist.

Wie oben beschrieben, eignen sich die erfindungsgemäßen Materialien ganz besonders als Elektrolumineszenzmaterialien in derart hergestellten PLEDs oder Displays.

Als Elektrolumineszenzmaterialien im Sinne der vorliegenden Erfindung gelten Materialien, die als aktive Schicht in einer PLED Verwendung finden können. Aktive Schicht bedeutet, dass die Schicht befähigt ist, bei Anlegen eines elektrischen Feldes Licht abzustrahlen (lichtemittierende Schicht) und/oder dass sie die Injektion und/oder den Transport der positiven und/oder negativen Ladungen verbessert (Ladungsinjektions-oder Ladungstransportschicht).

Gegenstand der vorliegenden Erfindung ist daher auch die Verwendung erfindungsgemäßer Polymere, Dendrimere oder Blends in einer PLED, insbesondere als Elektrolumineszenzmaterial.

Gegenstand der vorliegenden Erfindung ist somit ebenfalls eine PLED mit einer oder mehreren aktiven Schichten, wobei mindestens eine dieser aktiven Schichten ein oder mehrere erfindungsgemäße Polymere oder Dendrimere enthält. Die aktive Schicht kann beispielsweise eine lichtemittierende Schicht und/oder eine Transportschicht und/oder eine Ladungsinjektionsschicht sein.

Im vorliegenden Anmeldungstext und auch in den im Weiteren folgenden Beispielen wird auf die Verwendung erfindungsgemäßer Materialien in Bezug auf PLEDs und entsprechende Displays abgezielt. Trotz dieser Beschränkung der Beschreibung ist es für den Fachmann ohne weiteres erfinderisches Zutun möglich, die erfindungsgemäßen Materialien als Halbleiter auch für weitere Verwendungen in anderen elektronischen Devices (Vorrichtungen) zu benutzen, z.B. in organischen Feld-Effekt-Transistoren (OFETs), in organischen integrierten Schaltungen (O-ICs), in organischen Dünnfilmtransistoren (TFTs), in organischen Solarzellen (O-SCs), in organischen Laserdioden (O-Laser), in organischen photovoltaischen (OPV) Elementen oder Vorrichtungen oder in organischen Photorezeptoren (OPCs), um nur einige Anwendungen zu nennen.

Die Verwendung erfindungsgemäßer Materialien in den entsprechenden Vorrichtungen ist ebenfalls Gegenstand der vorliegenden Erfindung.

Ebenso ist es für den Fachmann ein Leichtes, die oben gemachten Beschreibungen für konjugierte Polymere ohne weiteres erfinderisches Zutun auf konjugierte Dendrimere zu übertragen. Solche konjugierten Dendrimere sind also auch Gegenstand der vorliegenden Erfindung.

Die folgenden Beispiele sollen die Erfindung erläutern, ohne sie einzuschränken. Insbesondere sind die darin beschriebenen Merkmale, Eigenschaften und Vorteile der dem betreffenden Beispiel zugrunde liegenden definierten Verbindungen auch auf andere, nicht im Detail aufgeführte, aber unter den Schutzbereich der Ansprüche fallende Verbindungen anwendbar, sofern an anderer Stelle nichts Gegenteiliges gesagt wird.

### Beispiel 1

Die Verbindung (1) wird wie folgt hergestellt

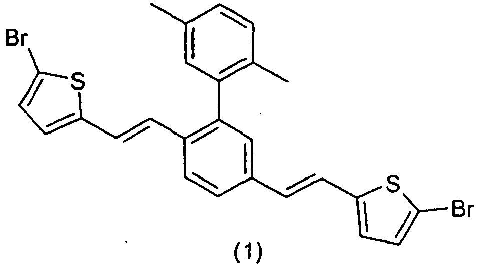

### 1.1 Herstellung von [5-(Diethoxy-phosphorylmethyl)-2',5'-dimethyl-biphenyl-2-ylmethyl]-phosphonsäurediethyl ester

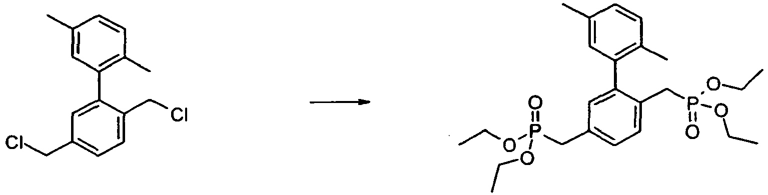

50 g (180 mmol) 2',5'-Bis-chloromethyl-2,5-dimethyl-biphenyl werden in 63 ml (366 mmol) Triethylphosphit bis zur Beendigung der Gasentwicklung auf 160°C erhitzt. Der verbleibende Rückstand wird ohne weitere Reinigung in die Folgereaktion eingesetzt.

### 1.2 Herstellung von 1,4-Bis(-2-brom-5-vinylthiophenyl)-2-(2,5-dimethylphenyl)-benzol

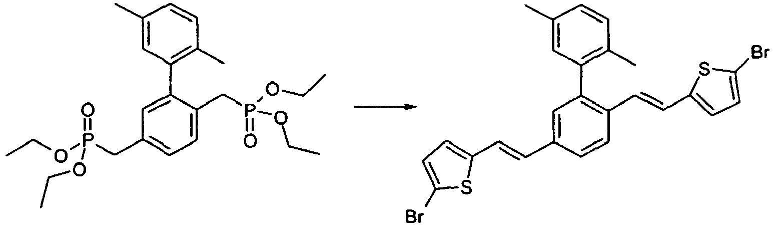

31,8 g (65,5 mmol) [5-(Diethoxy-phosphorylmethyl)-2',5'-dimethyl-biphenyl-2-ylmethyl]-phosphonsäure-diethylester werden in 225 ml DMF vorgelegt, unter Schutzgas bei ca. 5°C mit 25,18 g (262 mmol) NaO^{t}Bu versetzt und nach 30 Minuten Rührzeit bei 5°C Lösung von 25 g (131 mmol) 2-Bromthiophen-5-carbaldehyd in 60 ml DMF bei 5°C innerhalb 15 Minuten zugetropft. Nach 1 Stunde wird bei ca. 5°C 75 ml 4 M HCl zugetropft, der Niederschlag abgesaugt, mit MeOH gewaschen und getrocknet. Nach siebenmaliger Umkristallisation aus EtOH/Toluol (4 + 1) erhält man das Produkt in Form hellgelber Kristalle mit einer Reinheit von > 99,8 % (bestimmt durch RP-HPLC) und einer Ausbeute von 28,8 g (79%).

### Beispiel 2

Die Verbindung (2) wird wie folgt hergestellt

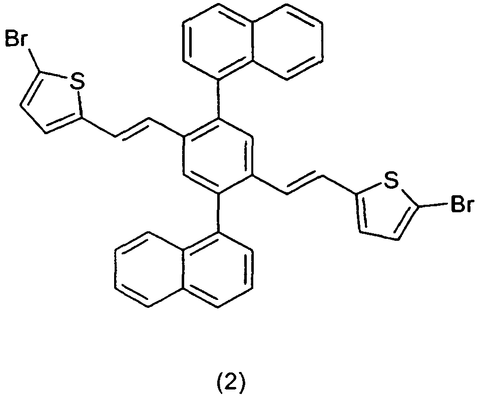

### 2.1 Herstellung von [4-(Diethoxy-phosphorylmethyl)-2,5-di-naphthalin-1-yl-benzyl]-phosphonsäurediethylester

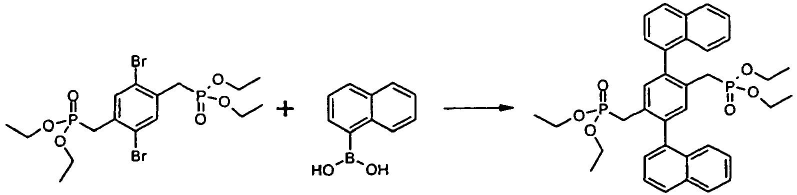

Eine Mischung aus 6,97 g (13 mmol) [2,5-Dibrom-4-(diethoxy-phosphorylmethyl)-benzyl]-phosphonsäurediethylester, 4,7 g (27,3 mmol) Naphthylboronsäure, 16,9 g (52 mmol) Cs₂CO₃ und 90 mg (0,4 mmol) Pd(OAc)₂ werden in 75 ml getrocknetem Dioxan suspendiert und 30 Minuten mit Stickstoff gesättigt. Im Anschluss werden 160 mg (0,8 mmol P(t-Bu)₃ zugegeben und für 2 Stunden zum Sieden erhitzt. Nach dem Abkühlen wird zwischen Wasser und Ethylacetat verteilt, die organische Phase mehrfach mit Wasser gewaschen, über Na₂SO₄ getrocknet und das Lösungsmittel im Vakuum entfernt. Nach Säulenchromatographie verbleiben 7,2 g (88%) des Diphosphonates als hellgelber Schaum.

### 2.2 Herstellung von 1,4-Bis(-2-brom-5-vinylthiophenyl)-2,5-(1-naphthyl)-benzol

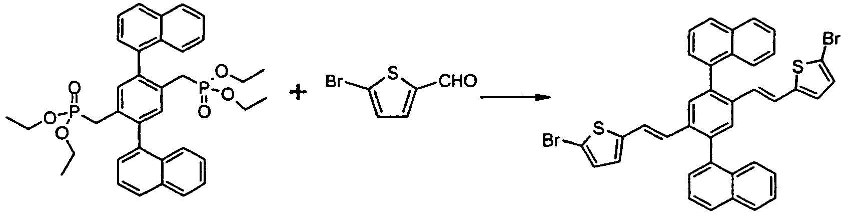

8,2 g (13mmol) Diphosphonat werden in 60 ml trockenem DMF gelöst und auf 5°C abgekühlt. Anschließend werden 5,0 g (52 mmol) NaO^{t}Bu in kleinen Portionen zugegeben, 5,5 g (28,6 mmol) 2-Bromthiophen-5-carboxaldehyd in 20 ml DMF zugetropft und 1 Stunde bei 5°C gerührt. Im Anschluss werden 25 ml 4 M HCl zugetropft, der Niederschlag abgesaugt, mit H₂O, Ethanol und Heptan gewaschen und aus Ethanol/Toluol (1 + 5) umkristallisiert. Man erhält das Produkt in Form gelber Kristalle mit einer Reinheit von > 99,9 % (RP-HPLC) und einer Ausbeute von 6,6 g (72 %).

### Beispiele 3 bis 7

Die erfindungsgemäßen Polymere P1 bis P4 sowie das Vergleichspolymer V1 werden durch SUZUKI-Kupplung gemäß WO 03/048225 synthetisiert. Die Zusammensetzung der synthetisierten Polymere **P1** bis **P4** sowie des Vergleichpolymers V1 ist wie folgt:

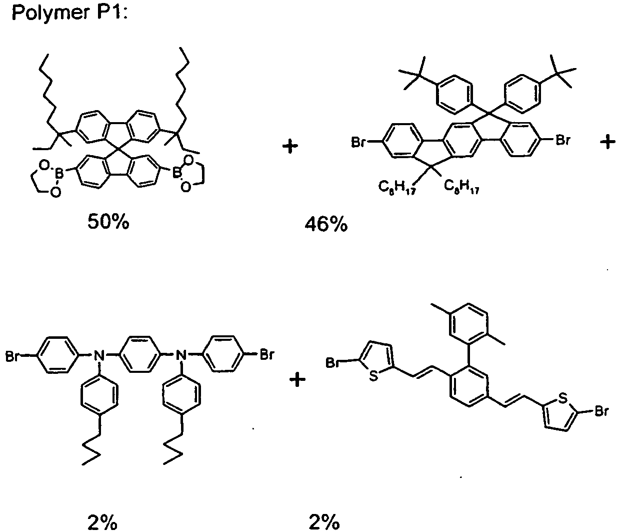

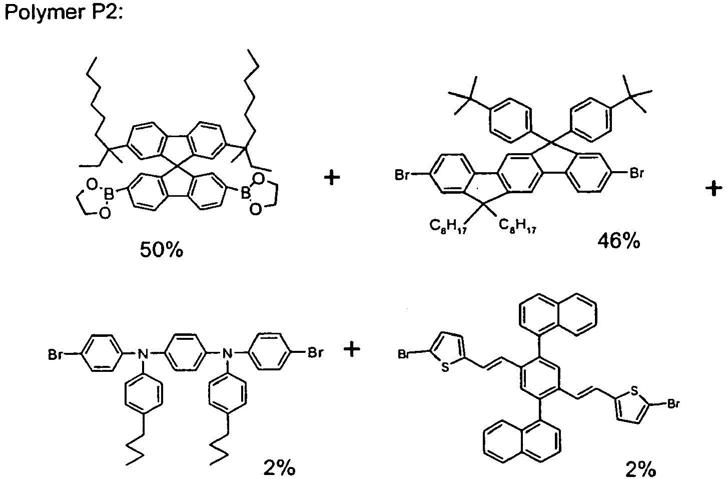

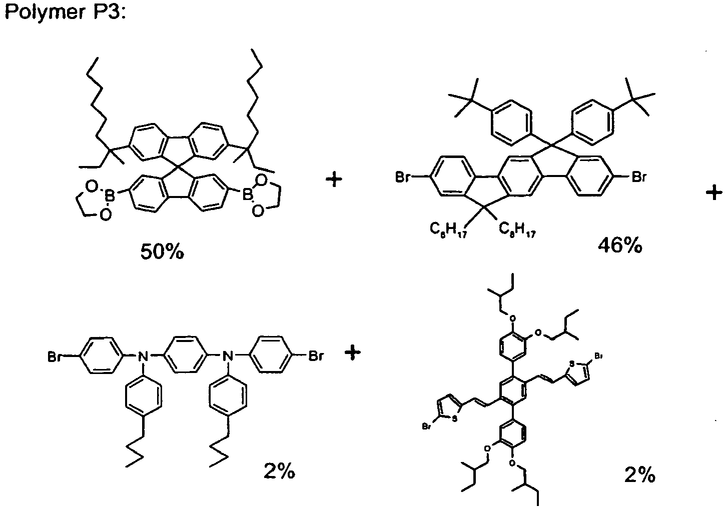

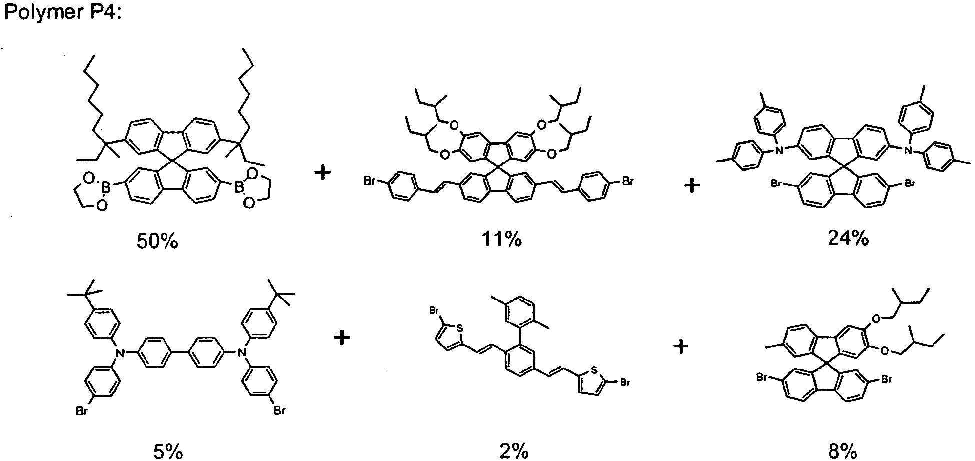

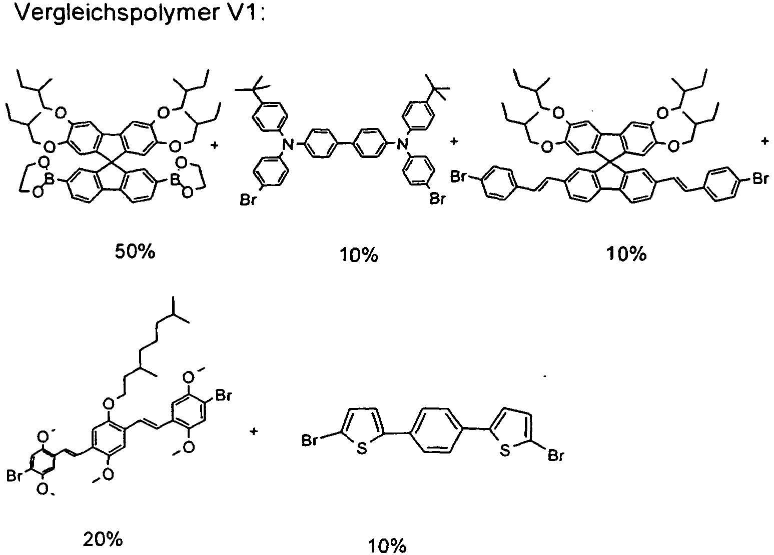

### Beispiel 8 bis 12: Device-Beispiele

### Herstellung einer PLED

Die Herstellung einer polymeren Leuchtdiode wird in der Patentliteratur bereits vielfach beschrieben (s. z.B. WO 04/037887). Um die vorliegende Erfindung beispielhaft zu erläutern, werden PLEDs mit den Polymeren P1 bis P4 sowie dem Vergleichspolymer V1 durch Spincoating auf zuvor mit PEDOT und einer lochinjizierenden Interlayer beschichtete ITO-Substrate hergestellt. (PEDOT ist ein Polythiophen-Derivat (Baytron P, von H. C. Starck, Goslar)). Die Schichtdicke der Polymerschicht ist ca. 80 nm. Danach wird eine Ba/Al-Kathode (Metalle von Aldrich) aufgedampft, die PLED verkapselt und elektrooptisch charakterisiert.

Die Ergebnisse, die bei Verwendung der Polymere P1 bis P4 sowie V1 in PLEDs erhalten werden, sind in Tabelle 1 zusammengefasst.

Wie man aus den Ergebnissen erkennen kann, ist die Effizienz der erfindungsgemäßen Polymere besser als die des Vergleichspolymers. Die Emissionsfarbe ist vergleichbar und die Lebensdauern sind dies berücksichtigend deutlich verbessert. Dies zeigt, dass die erfindungsgemäßen Polymere besser für den Einsatz in Displays geeignet sind als Polymere gemäß dem Stand der Technik.

| Bsp. | Polymer | Max.Eff [Cd/A] | U@100cd/m² [V] | CIE [x/y] | Lebensdauer [h] |
|---|---|---|---|---|---|
| 8 | P1 | 17,10 | 4,77 | 0,31/0,59 | 372@6000 |
| 9 | P2 | 17,38 | 4,89 | 0,32/0,60 | 159@6000 |
| 10 | P3 | 17,09 | 4,63 | 0,34/059 | 100@6000 |
| 11 | P4 | 8,98 | 3,17 | 0,33/0,60 | 560@2000" |
| 12 | V1 | 11,08 | 2,86 | 0,33/059 | 323@1500 |

| | | | | | |
|---|---|---|---|---|---|
| ^{a} MUX64, 100hz, kein Gegenpuls | | | | | |

## Patentansprüche

1. Verbindungen der Formel I 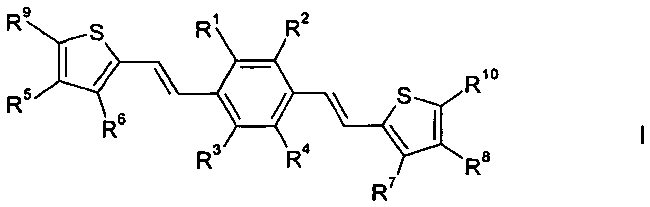 wobei die verwendeten Symbole und Indices folgende Bedeutung besitzen:
R¹ bis R¹⁰ sind unabhängig voneinander H, X¹, X¹-Sp-, -CN, -NO₂, -NCS, -NCO, -OCN, -SCN, -SF₅, -Si(R)₃ oder eine geradkettige, verzweigte oder cyclische Alkyl- oder Alkoxygruppe mit 1 bis 22 C-Atomen, in der auch ein oder mehrere nicht benachbarte C-Atome durch -C(R)=C(R)-, -C≡C-, -N(R)-, -O-, -S-, -CO-, -CO-O-, -O-CO-, -O-CO-O-ersetzt sein können, wobei auch ein oder mehrere H-Atome durch Fluor ersetzt sein können, eine Aryl-, Aryloxy-, Heteroaryl- oder Heteroaryloxygruppe mit 5 bis 40 C-Atomen, welche auch durch ein oder mehrere nicht-aromatische Reste R¹⁻¹⁰ substituiert sein kann, wobei auch zwei oder mehrere der Reste R¹⁻¹⁰ miteinander ein aliphatisches oder aromatisches, mono- oder polycyclisches Ringsystem bilden können, welches auch mit dem Benzolring oder den Thiophenringen in Formel I ein kondensiertes Ringsystem bilden kann, oder bedeuten eine Verknüpfung im Polymer,
R ist bei jedem Auftreten gleich oder verschieden H, Halogen, eine geradkettige, verzweigte oder cyclische Alkylkette mit 1 bis 22 C-Atomen, in der auch ein oder mehrere nicht benachbarte C-Atome durch -O-, -S-, -CO-, -CO-O-, -O-CO-, -O-CO-O- ersetzt sein können, wobei auch ein oder mehrere H-Atome durch Fluor ersetzt sein können, eine Aryl-, Aryloxy-, Heteroaryl- oder Heteroaryloxygruppe mit 5 bis 40 C-Atomen, welche auch durch ein oder mehrere nicht-aromatische Reste R¹⁻¹⁰ substituiert sein kann; dabei können auch mehrere Reste R miteinander und/oder mit weiteren Resten R¹⁻¹⁰ ein aromatisches oder aliphatisches, mono- oder polycyclisches Ringsystem bilden, welches auch mit dem Benzolring oder den Thiophenringen in Formel I ein kondensiertes Ringsystem bilden kann,
X¹ ist bei jedem Auftreten gleich oder verschieden eine reaktive Gruppe, ausgewählt aus Halogen, -OH, -COOH, -CY¹O, -CHO, -CH₂Cl, -N(R⁰)₂, -Si(R⁰)₃, -Sn(R⁰)₃, -B(R⁰)₂, -B(OR⁰)₂, -B(OH)₂, -CR⁰=C(R⁰)₂, -C≡CH, -O-SO₂R⁰, -SiMe₂F oder -SiMeF₂, worin Y¹ Halogen, Me Methyl und R⁰ Alkyl oder Aryl bedeutet, wobei auch zwei Gruppen R⁰ ein aromatisches oder aliphatisches, mono- oder polycyclisches Ringsystem bilden können, oder ausgewählt aus CH₂=CW¹-COO-, CH₂=CW¹-CO-, 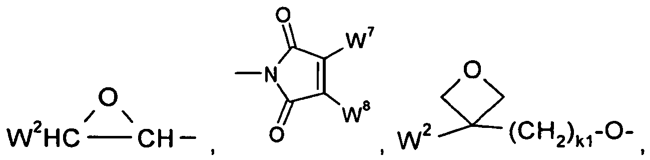 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W¹-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-, und W⁴W⁵W⁶Si-, worin W¹ H, F, Cl, CN, CF₃, Phenyl oder Alkyl mit 1 bis 5 C-Atomen bedeutet, W² und W³ jeweils unabhängig voneinander H oder Alkyl mit 1 bis 5 C-Atomen bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet und k₁ und k₂ jeweils unabhängig voneinander 0 oder 1 bedeuten,
Sp ist bei jedem Auftreten gleich oder verschieden eine Abstandsgruppe oder eine Einfachbindung, wobei die Abstandsgruppe Sp ausgewählt ist aus der Formel Sp'-X', so dass "X¹-Sp" "X¹-Sp'-X'-" bedeutet, wobei
Sp' Alkylen mit 1 bis 20 C-Atomen bedeutet, welches durch F, Cl, Br, I oder CN ein- oder mehrfach substituiert sein kann, und worin auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-, -NR⁰-CO-O-, -O-CO-NR⁰-, -NR⁰-CO-NR⁰-, -CH=CH- oder -C≡C- ersetzt sein können, dass O- und/oder S-Atome nicht direkt miteinander verknüpft sind,
X' -O-, -S-, -CO-, -COO-, -OCO-, -O-COO-, -CO-NR⁰-, -NR⁰-CO-, -NR⁰-CO-NR⁰-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=Cy³-, -C≡C-, -CH=CH-COO-, -OCO-CH=CH- oder eine Einfachbindung bedeutet,
R⁰ und R⁰⁰ jeweils unabhängig voneinander H oder Alkyl mit 1 bis 12 C-Atomen bedeuten, und
Y² und Y³ jeweils unabhängig voneinander H, F, Cl oder CN bedeuten,
worin mindestens einer der Reste R¹ bis R⁴ eine Arylgruppe bedeutet, welche durch ein oder mehrere nicht-aromatische Reste R¹⁻¹⁰ substituiert sein kann, wobei auch zwei oder mehrere der Reste R¹⁻¹⁰ miteinander ein aliphatisches oder aromatisches, mono- oder polycyclisches Ringsystem bilden können, welches auch mit dem Benzolring oder den Thiophenringen in Formel I ein kondensiertes Ringsystem bilden kann.

2. Verbindungen nach Anspruch 1, **dadurch gekennzeichnet, dass** R⁹ und R¹⁰ jeweils unabhängig voneinander X¹ bedeuten.

3. Verbindungen nach Anspruch 1 oder 2, **dadurch gekennzeichnet, dass** einer, zwei, drei oder vier der Reste R¹⁻⁴ eine Arylgruppe mit 5 bis 40 C-Atomen bedeuten, welche durch ein oder mehrere nicht-aromatische Reste R¹⁻¹⁰ substituiert sein kann, wobei auch zwei oder mehrere der Reste R¹⁻¹⁰ miteinander ein aliphatisches oder aromatisches, mono- oder polycyclisches Ringsystem bilden können, welches auch mit dem Benzolring oder den Thiophenringen in Formel I ein kondensiertes Ringsystem bilden kann, und die übrigen Reste R¹⁻⁴ H bedeuten.

4. Verbindungen nach Anspruch 3, **dadurch gekennzeichnet, dass** die von H verschiedenen Reste R¹⁻⁴ Phenyl oder 1-Naphthyl bedeuten, welche optional mit einem oder mehreren Resten L substituiert sind, wobei L ausgewählt ist aus F, Cl, Br, I, -CN, -NO₂, -NCO, -NCS, -OCN, -SCN, -C(=O)N(R)₂, -C(=O)Y¹, -C(=O)R, -N(R)₂, worin R die in Anspruch 1 angegebene Bedeutung hat und Y¹ Halogen bedeutet, Silyl, Aryl mit 4 bis 40 C Atomen, und geradkettigem oder verzweigtem Alkyl, Alkoxy, Alkylcarbonyl, Alkoxycarbonyl, Alkylcarbonlyoxy oder Alkoxycarbonyloxy mit 1 bis 22 C-Atomen, worin ein oder mehrere H-Atome gegebenfalls durch F oder Cl ersetzt sein können.

5. Verbindungen nach mindestens einem der Ansprüche 1 bis 4 ausgewählt aus folgenden Unterformeln 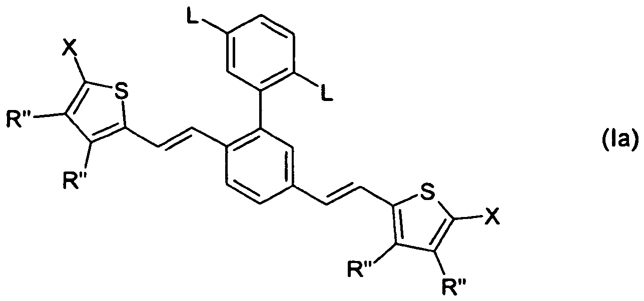 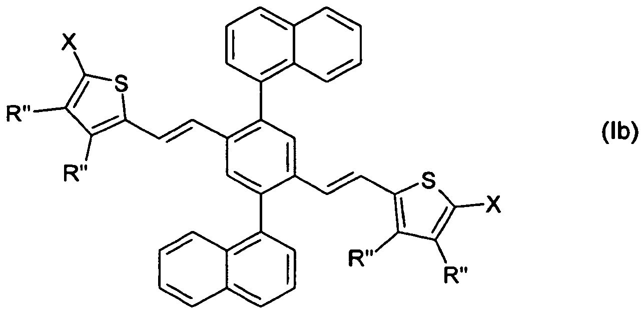 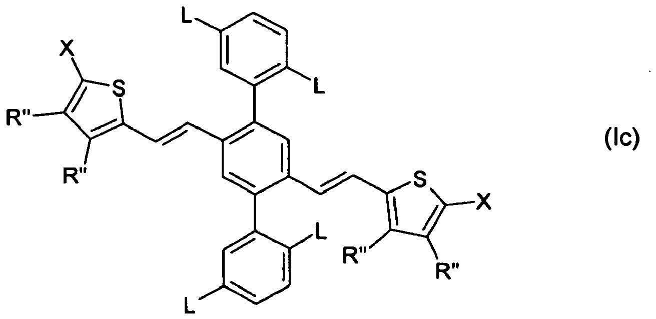 worin X eine der für X¹ in Anspruch 1 angegebenen Bedeutungen besitzt, R" eine der für R⁵⁻⁸ in Anspruch 1 angegebenen Bedeutungen besitzt und L jeweils unabhängig voneinander eine der in Anspruch 4 angegebenen Bedeutungen besitzt.

6. Polymere oder Dendrimere erhältlich aus einer oder mehreren Verbindungen nach mindestens einem der Ansprüche 1 bis 5.

7. Polymere oder Dendrimere nach Anspruch 6, **dadurch gekennzeichnet, dass** sie weitere Strukturelemente enthalten, ausgewählt aus den Gruppen der Fluorenylene, Spirobifluorenylene, Dihydrophenanthrenylene, Tetrahydropyrenylene, Stilbenylene, Bisstyrylarylene, 1,4-Phenylene, 1,4-Naphthylene, 1,4- oder 9,10-Anthrylene, 1,6- oder 2,7- oder 4,9-Pyrenylene, 3,9- oder 3,10-Perylenylene, 2,7- oder 3,6-Phenanthrenylene, 4,4'-Biphenylylene, 4,4"-Terphenylylene oder 4,4'-Bi-1,1'-naphthylylene.

8. Polymere oder Dendrimere nach Anspruch 6 oder 7, **dadurch gekennzeichnet, dass** sie weitere Strukturelemente enthalten, ausgewählt aus den Gruppen der Triarylamine, Triarylphosphine, Benzidine, Tetraarylen-para-phenylendiamine, Phenothiazine, Phenoxazine, Dihydrophenazine, Thianthrene, Dibenzo-p-dioxine, Phenoxathiine, Carbazole, Azulene, Thiophene, Pyrrole oder Furane.

9. Polymere oder Dendrimere nach mindestens einem der Ansprüche 6 bis 8, **dadurch gekennzeichnet, dass** sie weitere Strukturelemente enthalten, ausgewählt aus den Gruppen der Pyridine, Pyrimidine, Pyridazine, Pyrazine, Anthracene, Triarylborane, Oxadiazole, Chinoline, Chinoxaline oder Phenazine.

10. Polymere oder Dendrimere nach mindestens einem der Ansprüche 6 bis 9, **dadurch gekennzeichnet, dass** sie mindestens 50 mol% Einheiten nach Anspruch 7 und 2 bis 30 mol% Einheiten nach Anspruch 8 und/oder 9 enthalten.

11. Polymere oder Dendrimere nach mindestens einem der Ansprüche 6 bis 10, **dadurch gekennzeichnet, dass** der Anteil an Verbindungen der Formel I von 0,01 bis 25 mol% beträgt.

12. Blend enthaltend ein oder mehrerere Polymere oder Dendrimere nach mindestens einem der Ansprüche 6 bis 11, sowie optional eine oder mehrere weitere polymere, oligomere, dendritische oder niedermolekulare Substanzen.

13. Mischung bestehend aus niedermolekularen Verbindungen, enthaltend eine oder mehrere Verbindungen der Formel I nach mindestens einem der Ansprüche 1 bis 5 und zusätzlich eine oder mehrere lichtemittierende und/oder polymerisierbare Verbindungen.

14. Formulierung enthaltend ein oder mehrere Verbindungen, Polymere, Dendrimere, Blends oder Mischungen nach mindestens einem der Ansprüche 1 bis 13, sowie ein oder mehrere Lösungsmittel und/oder polymere Bindemittel.

15. Verwendung von Verbindungen, Polymeren, Dendrimeren, Blends, Mischungen oder Formulierungen nach mindestens einem der Ansprüche 1 bis 14 in einer elektrooptischen oder elektronischen Vorrichtung.

16. Vorrichtung enthaltend ein oder mehrere Verbindungen, Polymere, Dendrimere, Blends, Mischungen oder Formulierungen nach mindestens einem der Ansprüche 1 bis 14.

17. Vorrichtung nach Anspruch 16, **dadurch gekennzeichnet, dass** es sich um eine organische oder polymere organische Leuchtdiode (OLED, PLED), einen Feld-Effekt-Transistor (OFET), organischen Dünnfilmtransistor (O-TFT), eine organische integrierte Schaltung (O-IC), organische Solarzelle (O-SCs), organische Laserdiode (O-Laser), ein organisches photovoltaisches (OPV) Element oder Vorrichtung oder einen organischen Photorezeptor (OPC) handelt.

## Claims

1. Compounds of the formula I 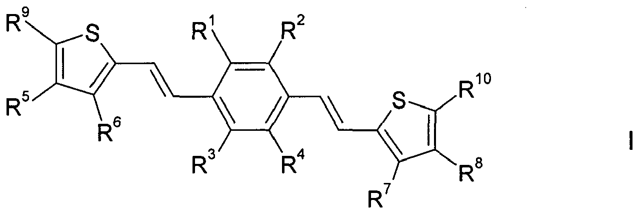 where the symbols and indices used have the following meaning:
R¹ to R¹⁰ are, independently of one another, H, X¹, X¹-Sp-, -CN, -NO₂, -NCS, -NCO, -OCN, -SCN, -SF₅, -Si(R)₃ or a straight-chain, branched or cyclic alkyl or alkoxy group having 1 to 22 C atoms, in which, in addition, one or more non-adjacent C atoms may be replaced by -C(R)=C(R)-, -C≡C-, -N(R)-, -O-, -S-, -CO-, -CO-O-, -O-CO-, -O-CO-O-, where, in addition, one or more H atoms may be replaced by fluorine, an aryl, aryloxy, heteroaryl or heteroaryloxy group having 5 to 40 C atoms, which may also be substituted by one or more non-aromatic radicals R¹⁻¹⁰, where, in addition, two or more of the radicals R¹⁻¹⁰ may form with one another an aliphatic or aromatic, mono- or polycyclic ring system, which may also form a condensed ring system with the benzene ring or the thiophene rings in formula I, or denote a link in the polymer,
R is on each occurrence, identically or differently, H, halogen, a straight-chain, branched or cyclic alkyl chain having 1 to 22 C atoms, in which, in addition, one or more non-adjacent C atoms may be replaced by -O-, -S-, -CO-, -CO-O-, -O-CO-, -O-CO-O-, where, in addition, one or more H atoms may be replaced by fluorine, an aryl, aryloxy, heteroaryl or heteroaryloxy group having 5 to 40 C atoms, which may also be substituted by one or more non-aromatic radicals R¹⁻¹⁰; a plurality of radicals R with one another and/or with further radicals R¹⁻¹⁰ may also form an aromatic or aliphatic, mono- or polycyclic ring system, which may also form a condensed ring system with the benzene ring or the thiophene rings in formula I,
X¹ is on each occurrence, identically or differently, a reactive group selected from halogen, -OH, -COOH, -CY¹O, -CHO, -CH₂Cl, -N(R⁰)₂, -Si(R⁰)₃, -Sn(R⁰)₃, -B(R⁰)₂, -B(OR⁰)₂, -B(OH)₂, -CR⁰=C(R⁰)₂, -C=CH, -O-SO₂R⁰, -SiMe₂F or -SiMeF₂, in which Y¹ denotes halogen, Me denotes methyl and R⁰ denotes alkyl or aryl, where, in addition, two groups R⁰ may form an aromatic or aliphatic, mono- or polycyclic ring system, or selected from CH₂=CW¹-COO-, CH₂=CW¹-CO-, 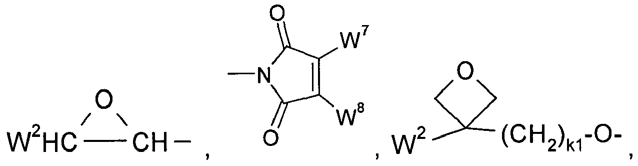 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-and W⁴W⁵W⁶Si-, in which W¹ denotes H, F, Cl, CN, CF₃, phenyl or alkyl having 1 to 5 C atoms, W² and W³ each, independently of one another, denote H or alkyl having 1 to 5 C atoms, W⁴, W⁵ and W⁶ each, independently of one another, denote Cl, oxaalkyl or oxacarbonylalkyl having 1 to 5 C atoms, W⁷ and W⁸ each, independently of one another, denote H, Cl or alkyl having 1 to 5 C atoms, Phe denotes 1,4-phenylene, and k₁ and k₂ each, independently of one another, denote 0 or 1,
Sp is on each occurrence, identically or differently, a spacer group or a single bond, where the spacer group Sp is selected from the formula Sp'-X', so that "X'-Sp-" denotes "X¹-Sp'-X'-", where
Sp' denotes alkylene having 1 to 20 C atoms, which may be mono- or polysubstituted by F, Cl, Br, I or CN and in which, in addition, one or more non-adjacent CH₂ groups may each be replaced, independently of one another, by -O-, -S-, -NH-, -NR⁰-, -SiR⁰R⁰⁰-, -CO-, -COO-, -OCO-, -OCO-O-, -S-CO-, -CO-S-, -NR⁰-CO-O-, -O-CO-NR⁰-, -NR⁰-CO-NR⁰-, -CH=CH- or -C≡C- in such a way that O and/or S atoms are not linked directly to one another,
X' denotes -O-, -S-, -CO-, -COO-, -OCO-, -O-COO-, -CO-NR⁰-, -NR⁰-CO-, -NR⁰-CO-NR⁰-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-COO-, -OCO-CH=CH- or a single bond,
R⁰ and R⁰⁰ each, independently of one another, denote H or alkyl having 1 to 12 C atoms, and
Y² and Y³ each, independently of one another, denote H, F, Cl or CN,
in which at least one of the radicals R¹ to R⁴ denotes an aryl group, which may be substituted by one or more non-aromatic radicals R¹⁻¹⁰, where, in addition, two or more of the radicals R¹⁻¹⁰ may form with one another an aliphatic or aromatic, mono- or polycyclic ring system, which may also form a condensed ring system with the benzene ring or the thiophene rings in formula I.

2. Compounds according to Claim 1, **characterised in that** R⁹ and R¹⁰ each, independently of one another, denote X¹.

3. Compounds according to Claim 1 or 2, **characterised in that** one, two, three or four of the radicals R¹⁻⁴ denote an aryl group having 5 to 40 C atoms, which may be substituted by one or more non-aromatic radicals R¹⁻¹⁰, where, in addition, two or more of the radicals R¹⁻¹⁰ may form with one another an aliphatic or aromatic, mono- or polycyclic ring system, which may also form a condensed ring system with the benzene ring or the thiophene rings in formula I, and the other radicals R¹⁻⁴ denote H.

4. Compounds according to Claim 3, **characterised in that** the radicals R¹⁻⁴ which are different from H denote phenyl or 1-naphthyl, each of which is optionally substituted by one or more radicals L, where L is selected from F, Cl, Br, I, -CN, -NO₂, -NCO, -NCS, -OCN, -SCN, -C(=O)N(R)₂, -C(=O)Y¹, -C(=O)R, -N(R)₂, in which R has the meaning indicated in Claim 1 and Y¹ denotes halogen, silyl, aryl having 4 to 40 C atoms, and straight-chain or branched alkyl, alkoxy, alkylcarbonyl, alkoxycarbonyl, alkylcarbonyloxy or alkoxycarbonyloxy having 1 to 22 C atoms, in which one or more H atoms may optionally be replaced by F or Cl.

5. Compounds according to at least one of Claims 1 to 4, selected from the following sub-formulae: 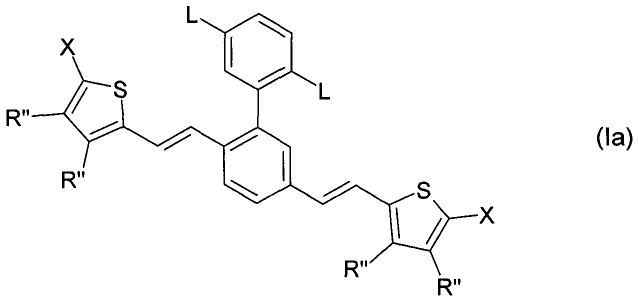 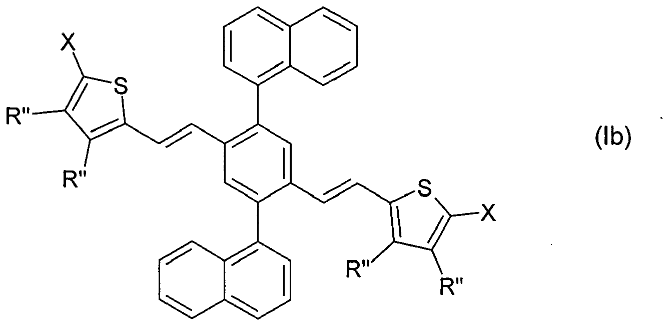 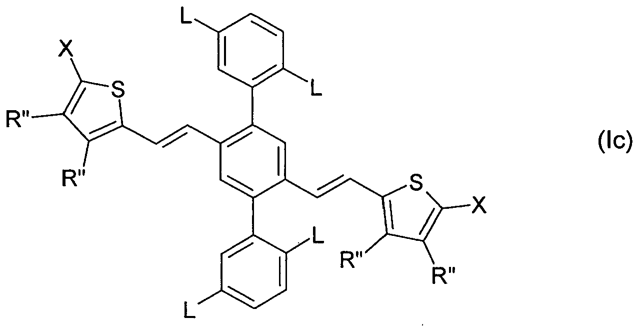 in which X has one of the meanings indicated for X¹ in Claim 1, R" has one of the meanings indicated for R⁵⁻⁸ in Claim 1, and L in each case, independently of one another, has one of the meanings indicated in Claim 4.

6. Polymers or dendrimers obtainable from one or more compounds according to at least one of Claims 1 to 5.

7. Polymers or dendrimers according to Claim 6, **characterised in that** they contain further structural elements selected from the groups of the fluorenylenes, spirobifluorenylenes, dihydrophenanthrenylenes, tetrahydropyrenylenes, stilbenylenes, bisstyrylarylenes, 1,4-phenylenes, 1,4-naphthylenes, 1,4- or 9,10-anthrylenes, 1,6- or 2,7- or 4,9-pyrenylenes, 3,9- or 3,10-perylenylenes, 2,7- or 3,6-phenanthrenylenes, 4,4'-biphenylylenes, 4,4"-terphenylylenes or 4,4'-bi-1,1'-naphthylylenes.

8. Polymers or dendrimers according to Claim 6 or 7, **characterised in that** they contain further structural elements selected from the groups of the triarylamines, triarylphosphines, benzidines, tetraarylene-paraphenylenediamines, phenothiazines, phenoxazines, dihydrophenazines, thianthrenes, dibenzo-p-dioxins, phenoxathiynes, carbazoles, azulenes, thiophenes, pyrroles or furans.

9. Polymers or dendrimers according to at least one of Claims 6 to 8, **characterised in that** they contain further structural elements selected from the groups of the pyridines, pyrimidines, pyridazines, pyrazines, anthracenes, triarylboranes, oxadiazoles, quinolines, quinoxalines or phenazines.

10. Polymers or dendrimers according to at least one of Claims 6 to 9, **characterised in that** they contain at least 50 mol% of units according to Claim 7 and 2 to 30 mol% of units according to Claim 8 and/or 9.

11. Polymers or dendrimers according to at least one of Claims 6 to 10, **characterised in that** the proportion of compounds of the formula I is 0.01 to 25 mol%.

12. Blend comprising one or more polymers or dendrimers according to at least one of Claims 6 to 11 and optionally one or more further polymeric, oligomeric, dendritic or low-molecular-weight substances.

13. Mixture consisting of low-molecular-weight compounds, comprising one or more compounds of the formula I according to at least one of Claims 1 to 5 and additionally one or more light-emitting and/or polymerisable compounds.

14. Formulation comprising one or more compounds, polymers, dendrimers, blends or mixtures according to at least one of Claims 1 to 13 and one or more solvents and/or polymeric binders.

15. Use of compounds, polymers, dendrimers, blends, mixtures or formulations according to at least one of Claims 1 to 14 in an electro-optical or electronic device.

16. Device comprising one or more compounds, polymers, dendrimers, blends, mixtures or formulations according to at least one of Claims 1 to 14.

17. Device according to Claim 16, **characterised in that** it is an organic or polymeric organic light-emitting diode (OLED, PLED), field-effect transistor (OFET), organic thin-film transistor (O-TFT), organic integrated circuit (O-IC), organic solar cell (O-SC), organic laser diode (O-laser), organic photovoltaic (OPV) element or device or organic photoreceptor (OPC).

## Revendications

1. Composés de la formule I 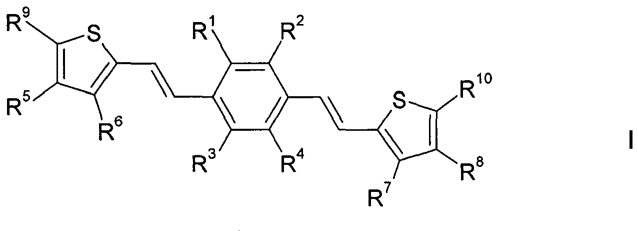 dans laquelle les symboles et indices utilisés présentent la signification qui suit:
R¹ à R¹⁰ sont, indépendamment les uns des autres, H, X¹, X¹-Sp-, -CN, -NO₂, -NCS, -NCO, -OCN, -SCN, -SF₅, -Si(R)₃ ou un groupe alkyle ou alcoxy en chaîne droite, ramifié ou cyclique comportant de 1 à 22 atomes de C, où, en outre, un ou plusieurs atomes de C non adjacents peuvent être remplacés par -C(R)=C(R)-, -C≡C-, -N(R)-, -O-, -S-, -CO-, -CO-O-, -O-CO-, -O-CO-O-, où, en outre, un ou plusieurs atomes de H peut/peuvent être remplacé(s) par fluor, un groupe aryle, aryloxy, hétéroaryle ou hétéroaryloxy comportant de 5 à 40 atomes de C, lequel peut également être substitué par un ou plusieurs radicaux non aromatiques R¹⁻¹⁰, où, en outre, deux des radicaux R¹⁻¹⁰ ou plus peuvent former l'un avec l'autre ou les uns avec les autres un système de cycle mono- ou polycyclique aliphatique ou aromatique, lequel peut également former un système de cycle condensé avec le cycle benzène ou les cycles thiophène dans la formule I, ou représentent une liaison dans le polymère,
R est, pour chaque occurrence, de manière identique ou différente, H, halogène, une chaîne alkyle en chaîne droite, ramifié ou cyclique comportant de 1 à 22 atomes de C, où, en outre, un ou plusieurs atomes de C non adjacents peut/peuvent être remplacé(s) par -O-, -S-, -CO-, -CO-O-, -O-CO-, -O-CO-O-, où, en outre, un ou plusieurs atomes de H peut/peuvent être remplacé(s) par fluor, un groupe aryle, aryloxy, hétéroaryle ou hétéroaryloxy comportant de 5 à 40 atomes de C, lequel peut également être substitué par un ou plusieurs radicaux non aromatiques R¹⁻¹⁰ ; une pluralité de radicaux R, les uns avec les autres et/ou avec d'autres radicaux R¹⁻¹⁰, peuvent également former un système de cycle mono- ou polycyclique aromatique ou aliphatique, lequel peut également former un système de cycle condensé avec le cycle benzène ou les cycles thiophène dans la formule I,
X¹ est, pour chaque occurrence, de manière identique ou différente, un groupe réactif choisi parmi halogène, -OH, -COOH, -CY¹O, -CHO, -CH₂Cl, -N(R⁰)₂, -Si(R⁰)₃, -Sn(R⁰)₃, -B(R⁰)₂, -B(OR⁰)₂, -B(OH)₂, -CR⁰=C(R⁰)₂, -C≡CH, -O-SO₂R⁰, -SiMe₂F ou -SiMeF₂, où Y¹ représente halogène, Me représente méthyle et R⁰ représente alkyle ou aryle, où, en outre, deux groupes R⁰ peuvent former un système de cycle mono- ou polycyclique aromatique ou aliphatique, ou choisi parmi CH₂=CW¹-COO-, CH₂=CW¹-CO-, 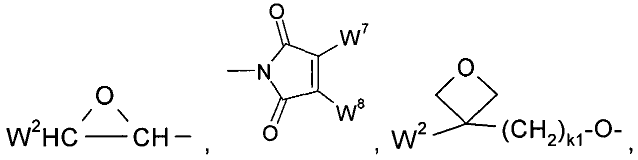 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-et W⁴W⁵W⁶Si-, où W¹ représente H, F, Cl, CN, CF₃, phényle ou alkyle comportant de 1 à 5 atomes de C, W² et W³, chacun indépendamment de l'autre, représentent H ou alkyle comportant de 1 à 5 atomes de C, W⁴, W⁵ et W⁶, chacun indépendamment des autres, représentent Cl, oxaalkyle ou oxacarbonylalkyle comportant de 1 à 5 atomes de C, W⁷ et W⁸, chacun indépendamment de l'autre, représentent H, Cl ou alkyle comportant de 1 à 5 atomes de C, Phe représente 1,4-phénylène, et k₁ et k₂, chacun indépendamment de l'autre, représentent 0 ou 1,
Sp est, pour chaque occurrence, de manière identique ou différente, un groupe d'espaceur ou une liaison simple, où le groupe d'espaceur Sp est choisi parmi la formule Sp'-X', de telle sorte que "X¹-Sp" représente "X¹-Sp'-X'-", où
Sp' représente alkylène comportant de 1 à 20 atomes de C, lequel peut être mono- ou polysubstitué par F, Cl, Br, I ou CN et où, en outre, un ou plusieurs groupes CH₂ non adjacents peuvent chacun être remplacés, indépendamment les uns des autres, par -O-, -S-, -NH-, -NR⁰-, -SiR⁰R⁰⁰-, -CO-, -COO-, -OCO-, -OCO-O-, -S-CO-, -CO-S-, -NR⁰-CO-O-, -O-CO-NR⁰-, -NR⁰-CO-NR⁰-, -CH=CH- ou -C=C- de telle sorte que des atomes de O et/ou de S ne soient pas liés directement les uns aux autres,
X' représente -O-, -S-, -CO-, -COO-, -OCO-, -O-COO-, -CO-NR⁰-, -NR⁰-CO-, -NR⁰-CO-NR⁰-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C- -CH=CH-COO-, -OCO-CH=CH- ou une liaison simple,
R⁰ et R⁰⁰ représentent, chacun indépendamment de l'autre, H ou alkyle comportant de 1 à 12 atomes de C, et
Y² et Y³ représentent, chacun indépendamment de l'autre, H, F, Cl ou CN,
où au moins l'un des radicaux R¹ à R⁴ représente un groupe aryle, lequel peut être substitué par un ou plusieurs radicaux non aromatiques R¹⁻¹⁰, où, en outre, deux des radicaux R¹⁻¹⁰ ou plus peuvent former l'un avec l'autre ou les uns avec les autres un système de cycle mono- ou polycyclique aliphatique ou aromatique, lequel peut également former un système de cycle condensé avec le cycle benzène ou les cycles thiophène dans la formule I.

2. Composés selon la revendication 1, **caractérisés en ce que** R⁹ et R¹⁰ représentent, chacun indépendamment de l'autre, X¹.

3. Composés selon la revendication 1 ou 2, **caractérisés en ce qu'**un, deux, trois ou quatre des radicaux R¹⁻⁴ représentent un groupe aryle comportant de 5 à 40 atomes de C, lequel peut être substitué par un ou plusieurs radicaux non aromatiques R¹⁻¹⁰, où, en outre, deux des radicaux R¹⁻¹⁰ ou plus peuvent former l'un avec l'autre ou les uns avec les autres un système de cycle mono- ou polycyclique aliphatique ou aromatique, lequel peut également former un système de cycle condensé avec le cycle benzène ou les cycles thiophène dans la formule I, et les autres radicaux R¹⁻⁴ représentent H.

4. Composés selon la revendication 3, **caractérisés en ce que** les radicaux R¹⁻⁴ qui sont différents de H représentent phényle ou 1-naphtyle, dont chacun est en option substitué par un ou plusieurs radicaux L, où L est choisi parmi F, Cl, Br, I, -CN, -NO₂, -NCO, -NCS, -OCN, -SCN, -C(=O)N(R)₂, -C(=O)Y¹, -C(=O)R, -N(R)₂, où R présente la signification indiquée selon la revendication 1 et Y¹ représente halogène, silyle, aryle comportant de 4 à 40 atomes de C, et alkyle, alcoxy, alkylcarbonyle, alcoxycarbonyle, alkylcarbonyloxy ou alcoxycarbonyloxy en chaîne droite ou ramifié comportant de 1 à 22 atomes de C, où un ou plusieurs atomes de H peut/peuvent en option être remplacé(s) par F ou Cl.

5. Composés selon au moins l'une des revendications 1 à 4, choisis parmi les sous-formules qui suivent : 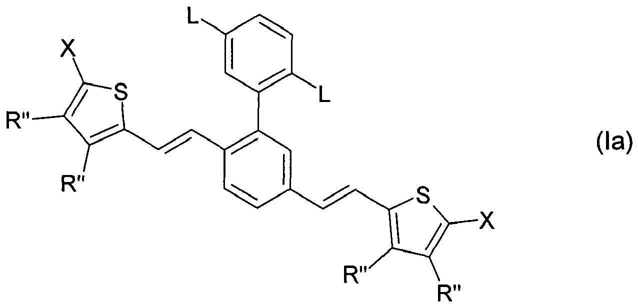 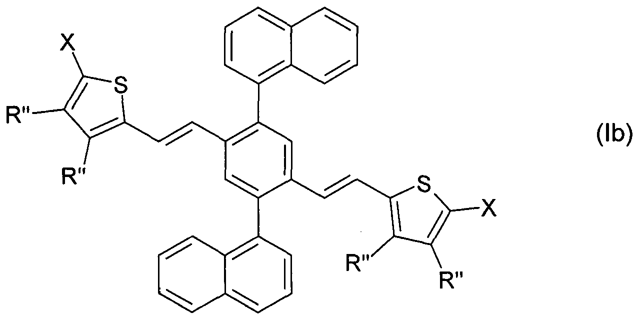 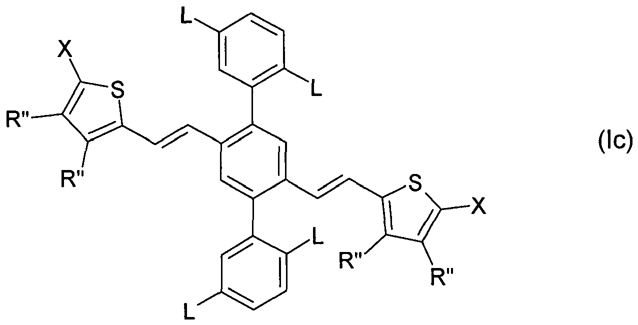 dans lesquelles X présente l'une des significations indiquées pour X¹ selon la revendication 1, R" présente l'une des significations indiquées pour R⁵⁻⁸ selon la revendication 1, et L présente, dans chaque cas indépendamment des autres, l'une des significations indiquées selon la revendication 4.

6. Polymères ou dendrimères pouvant être obtenus à partir d'un ou de plusieurs composés selon au moins l'une des revendications 1 à 5.

7. Polymères ou dendrimères selon la revendication 6, **caractérisés en ce qu'**ils contiennent des éléments structurels supplémentaires choisis parmi les groupes des fluorénylènes, spirobifluorénylènes, dihydrophénanthrénylènes, tétrahydropyrénylènes, stilbénylènes, bisstyrylarylènes, 1,4-phénylènes, 1,4-naphtylènes, 1,4- ou 9,10-anthrylènes, 1,6- ou 2,7- ou 4,9-pyrénylènes, 3,9- ou 3,10-perylénylènes, 2,7- ou 3,6-phénanthrénylènes, 4,4'-biphénylylènes, 4,4"-terphénylylènes ou 4,4'-bi-1,1'-naphtylylènes.

8. Polymères ou dendrimères selon la revendication 6 ou 7, **caractérisés en ce qu'**ils contiennent des éléments structurels supplémentaires choisis parmi les groupes des triarylamines, triarylphosphines, benzidines, tétraarylène-para-phénylènediamines, phénothiazines, phénoxazines, dihydrophénazines, thianthrènes, dibenzo-p-dioxines, phénoxathiynes, carbazoles, azulènes, thiophènes, pyrroles ou furannes.

9. Polymères ou dendrimères selon au moins l'une des revendications 6 à 8, **caractérisés en ce qu'**ils contiennent des éléments structurels supplémentaires choisis parmi les groupes des pyridines, pyrimidines, pyridazines, pyrazines, anthracènes, triarylboranes, oxadiazoles, quinolines, quinoxalines ou phénazines.

10. Polymères ou dendrimères selon au moins l'une des revendications 6 à 9, **caractérisés en ce qu'**ils contiennent au moins 50 mol% d'unités selon la revendication 7 et 2 à 30 mol% d'unités selon la revendication 8 et/ou 9.

11. Polymères ou dendrimères selon au moins l'une des revendications 6 à 10, **caractérisés en ce que** la proportion de composés de la formule I est de 0,01 à 25 mol%.

12. Coupage comprenant un ou plusieurs polymères ou dendrimères selon au moins l'une des revendications 6 à 11 et en option, une ou plusieurs autres substances polymériques, oligomériques, dendritiques ou de poids moléculaire faible.

13. Mélange constitué par des composés de poids moléculaire faible, comprenant un ou plusieurs composés de la formule I selon au moins l'une des revendications 1 à 5 et additionnellement, un ou plusieurs composés émetteurs de lumière et/ou polymérisables.

14. Formulation comprenant un ou plusieurs composés, polymères, dendrimères, coupages ou mélanges selon au moins l'une des revendications 1 à 13 et un ou plusieurs solvants et/ou liants polymériques.

15. Utilisation de composés, polymères, dendrimères, coupages, mélanges ou formulations selon au moins l'une des revendications 1 à 14 dans un dispositif électro-optique ou électronique.

16. Dispositif comprenant un ou plusieurs composés, polymères, dendrimères, coupages, mélanges ou formulations selon au moins l'une des revendications 1 à 14.

17. Dispositif selon la revendication 16, **caractérisé en ce qu'**il s'agit d'une diode émettrice de lumière organique ou polymérique (OLED, PLED), d'un transistor à effet de champ (OFET), d'un transistor à film mince organique (O-TFT), d'un circuit intégré organique (O-IC), d'une cellule solaire organique (O-SC), d'une diode laser organique (O-laser), d'un élément ou dispositif photovoltaïque organique (OPV) ou d'un photorécepteur organique (OPC).
